(19) Europäisches Patentamt
European Patent Office
Office européen des brevets

(11) **EP 2 249 889 B1**

(12) **EUROPEAN PATENT SPECIFICATION**

(45) Date of publication and mention
of the grant of the patent:
**13.08.2014 Bulletin 2014/33**

(21) Application number: **09707345.6**

(22) Date of filing: **05.02.2009**

(51) Int Cl.:
***A61L 27/30*** *(2006.01)*    ***A61L 27/56*** *(2006.01)*

(86) International application number:
**PCT/IB2009/050483**

(87) International publication number:
**WO 2009/098658 (13.08.2009 Gazette 2009/33)**

(54) **CRYSTALLINE SURGICAL IMPLANT COMPOSITE MATERIALS AND KITS AND METHODS OF MANUFACTURE**

VERBUNDMATERIALIEN UND KITS FÜR EIN KRISTALLINES CHIRURGISCHES IMPLANTAT SOWIE VERFAHREN ZU IHRER HERSTELLUNG

MATÉRIAUX COMPOSITES D'IMPLANTS CHIRURGICAUX CRISTALLINS ET KITS ET PROCÉDÉS DE FABRICATION CORRESPONDANTS

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **07.02.2008 US 26764 P**
**22.08.2008 US 90994 P**

(43) Date of publication of application:
**17.11.2010 Bulletin 2010/46**

(73) Proprietor: **Sandvik Intellectual Property AB**
**811 81 Sandviken (SE)**

(72) Inventors:
• **STRÖMME, Maria**
**756 51 Uppsala (SE)**
• **ENGQVIST, Håkan**
**742 36 Östhammar (SE)**

(56) References cited:
**WO-A1-2008/056323    US-A1- 2003 099 762**
**US-A1- 2003 175 444**

Description

## FIELD OF THE INVENTION

The present invention is directed to composite materials, for example, surgical implant composite materials, kits including such composite materials, and methods of manufacturing such composite materials, wherein the composite materials have a crystalline outer surface with a grain size greater than 1 nm.

## BACKGROUND OF THE INVENTION

Metallic implants such as titanium, titanium alloys, stainless steel and Co-Cr alloys are widely used as surgical implants owing to their mechanical strength, biocompatibility, chemical inertness and machinability. Examples of metallic implants include dental implants, cochlear implants, pedicle screws, spinal implants, hip implants, plates, nails, arm and leg amputation implants, and the like. Non-resorbable ceramic implants are also used owing to their mechanical strength, biocompatibility, chemical inertness, and superior aesthetics and high wear resistance. Examples of non-resorbable ceramic implant materials include zirconium dioxide and aluminum oxide. For an overview description of implant materials and their use, reference is made to Ratner et al., Biomaterials Science; An Introduction to Material in Medicine, 2nd Edition, San Diego (2004).

One of the requirements of implants, wherever they are used in the body, is the ability to form a mechanically stable unit with the surrounding hard or soft tissue. An unstable unit may function less efficiently or cease functioning completely, and/or may induce an excessive tissue response. This may cause patient discomfort and, in certain situations, an unstable implant may need to be surgically removed, for example, owing to little or no tissue in growth to the implant, infection, and/or weak bone formation due to osteoporosis or other diseases.

Several methods have been proposed in the literature to overcome instability owing to insufficient host tissue in growth. The roughness, morphology and/or chemical composition of the implant surface can be modified to promote tissue in growth. For an overview of the state-of-the-art of surface modification of surgical implants, reference is made to Brunette et al, Titanium is Medicine, Heidelberg (2001) and Ellingsen et al, Bio-Implant Interface: Improving Biomaterials and Tissue Reaction, CRC Press (2003). The surface roughness of implants is typically controlled via acid etching and/or grit blasting of the surface; see, for example, U.S. Patent No. 6,491,723 and Brunette et al. A roughness of 1 to 5 micrometers has been proven to promote bone in growth. Also, heating of the surface using a focused laser has been proposed to create an appropriate surface roughness at specific locations.

A combination of improved surface roughness, morphology and modified composition of titanium implants has been obtained via anodic oxidation of an implant, see WO 2005/055860 and U.S. Patent No. 6,183,255. The anodic oxidation results in a several micrometer thick porous titanium oxide surface layer, see Lin et al, "Corrosion test, cell behavior test, and in vivo study of gradient TiO2 layers produced by compound electrochemical oxidation," Journal of Biomedical Materials Research, Part A, 78(3):515-22 (2006 Sep 1). The oxide has been proposed to be used as a carrier of bone morphogenic proteins and peptides to promote bone formation on the implant; see also SE 523,012. It has also been shown that $TiO_{2-x}$ films (with x between 0 and 0.35), possibly containing implanted H, Ta or Nb, are blood compatible, and that this type of material can be produced by a plasma immersion ion implantation process. See U.S. Patent Publication No. 2003/0175444 A1, which describes a gradient structure from TiN at the implant interface to $TiO_{2-x}$ with a rutile structure at the surface. The TiN layer is added to increase the mechanical properties of the coating. The addition of H, Ta or Nb to the coating is also proposed to be performed using a sputtering method.

Methods to promote bone formation via tailoring the chemical composition of the implant often involve coating of the metal or ceramic surgical implant with a ceramic layer consisting essentially of hydroxyapatite (HA) or calcium phosphate; see Yang et al, "A review on calcium phosphate coatings produced using a sputtering process-an alternative to plasma spraying," Biomaterials, 26:327-337 (2005). HA is a commonly used bioactive ceramic material and its use is predicated upon its excellent affinity to bone. This property is due to the fact that HA is a major component of the bones and teeth of living animals. Bioactivity, in this context, is defined as interfacial bonding of an implant to tissue by means of formation of a biologically active hydroxyapatite layer on the implant surface, Ducheyne et al, "Bioceramics: material characteristics versus in vivo behaviour," Annuls of the New York Academy of Science, 523 (June 10, 1988). Bioactive materials have been proven to promote bone formation and to form a stable bond to bone, Hench, "Biomaterials: a forecast for the future," Biomaterials, 19:1419-14239 (1998). A bioactive material may spontaneously form a layer of HA on a surface in vivo when it comes in contact with body fluids. Synthetically produced HA also has bioactive properties and a new layer of carbon rich HA forms on its surface when implanted. Prediction of a material's bioactivity can be made both in vivo and in vitro, Kokubo et al, "How useful is SBF in predicting in vivo bone bioactivity," Biomaterials, 27:2907-2915 (2006). US2003/099762 discloses an implant comprising a first layer having a first thermal expansion coefficient and comprising an oxide compound (e.g. $Al_2O_3$, $TiO_2$, $ZrO_2$ etc. and combinations thereof), and a second layer comprising an apatite (Ca phosphate) having greater than 90% crystallinity and a binder. The apatite and the apatite-binder is present in a gradient perpendicular to the implant surface. The multilayer coating can comprise a third layer comprising an active agent. The third layer is preferably less than 70% crystalline. The lower crystallinity in the third layer as compared to the second layer can facilitate the integration of the implant into the surrounding tissue.

In addition to HA and calcium phosphate, a number of other material systems have been shown to be bioactive, e.g. Wollastonite (calcium silicate), see De Aza et al, "Bioactivity of pseudowollastonite in human saliva," Journal of Dentistry, 27:107-113 (1999); Bioglass, see Hench et al; and Rutile (titanium dioxide) and Anatase (titanium dioxide), see Kim, "Ceramic bioactivity and related biomimetic strategy," Currant Opinion in Solid State & Materials Science, 7:289-299 (2003). Other methods to obtain bioactivity of titanium implants are via chemical treatment in Na-F, see U.S. Patent No. 5,571,188, and NaOH, see Ma et al, "Biomimetic processing of nanocrystallite bioactive apatite coating on titanium," Nanotechnology, 14:619-623 (2003).

In summary, techniques that have been proposed to obtain a surface composition that promotes bone in growth include:

1. Coating of the surface with HA or calcium phosphate or other bioactive materials. However, difficulties in the coating process result in low coating adhesion or coating stability, see U.S. Patent No. 5,480,438, often due to technical difficulties in depositing thin films of complex oxide systems.

2. Chemical treatment of Ti with NaOH or Na-F. These surfaces can then be implanted directly or after soaking in simulated body fluid or phosphate buffered saline to form an "HA layer on the surface; however, the chemical treatment is only suitable for Ti metals.

3. Heat treatment of Ti to form. crystalline anatase or rutile, which may then be implanted. However, to achieve crystalline titanium oxide, the treatment temperature needs to be in excess of 400°C, resulting in reduced mechanical strength of the implant. Additionally, this treatment is only suitable for Ti metals.

4. Anodic oxidation of Ti to form anatase or rutile or a mixture thereof.

5. Deposition of a Ti oxide-containing layer on the implant base, forming a coating containing crystalline grains of anatase or rutile or combinations thereof. When the deposition is performed with substrate temperatures below 400°C, the grains size is usually below 10 nm depending on the coating parameters; see Zhou et al, "Plasma-controlled nanocrystallinity and phase composition of TiO2: a smart way to enhance biomimetic response," J. Biomedical Materials Research, 81A:453-464 (2007).

It has also been proposed that a drug can be encapsulated into a resorbable polymer which is then coated on a metallic implant to allow for drug delivery from the implant surface, see WO 2006/107336. The release kinetics are controlled via the polymer composition, thickness and drug loading. Methods to immobilize bio molecules on implants via amide cross-linking have also been described, see U.S. Patent Publication No. 2006/0193968.

It has previously been shown that in titanium dioxide-containing coatings made by sol gel methods, a larger crystallite size enhances the bioactivity of the coating, and that annealing titanium dioxide-containing sol gel films at temperatures above 500 °C increases the crystalline grain size. See, Rossi et al, "Comparison between sol-gel-derived anatase- and rutile-structured TiO2 coatings in soft-tissue environment," J. Biomedical Materials Research, 82A:965-974

(2007). However, for a broad range of substrate materials including, but not limited to, substrates containing titanium which are commonly used in surgical implants, exposure to temperatures higher than about 400-500°C is generally avoided in order avoid compromising the mechanical stability of the substrate.

Accordingly, there is a continuing need for improved implant materials and implants to provide improved implant use.

## SUMMARY OF THE INVENTION

The invention is directed to methods of manufacturing composite materials. In one embodiment, a method of forming a surgical implant composite material comprises depositing a thin film coating on a surgical implant substrate, the thin film coating comprising $TiO_{2-x}M_y$, wherein M is one or more elements which does not adversely effect adherence of the coating to the substrate, y is the sum of the mols of all M elements, $0 \leq x < 2$ and $0 \leq y \leq 1$, and wherein an outermost portion of the thin film coating is crystalline, and heating the deposited thin film coating at a temperature sufficiently high to increase the crystalline grain size in the thin film coating and further comprising a step of forming a biomimetic coating comprising calcium phosphate on the thin film coating by a biomimetic precipitation from a buffer solution comprising calcium phosphate.

The invention is directed to a method of preparing a surgical implant composite material for implantation in an individual. The method comprises selecting a releasable agent to be loaded on the surgical implant composite material based on a condition of the individual, and contacting the surgical implant composite material with a solution of the releasable agent to load the releasable agent onto the surgical implant composite material, the surgical implant composite material comprising a surgical implant substrate and a thin film coating deposited on the substrate, the thin film coating comprising $TiO_{2-x}M_y$, wherein M is one or more elements which does not adversely effect adherence of the coating to the substrate, y is the sum of the mols of all M elements, $0 \leq x < 2$ and $0 \leq y \leq 1$, and wherein an outermost portion of the thin film coating is crystalline, with crystalline grains larger than 1 nm, wherein the forming of the surgical implant composite material further comprises a step of forming a biomimetic coating comprising calcium phosphate on the thin film coating by a biomimetic precipitation from a buffer solution comprising calcium phosphate and wherein the releasable agent is loaded onto the biomimetic coating and the releasable agent comprising an active pharmaceutical ingredient, ion or bio molecule, or a combination thereof.

Additional embodiments of the invention, along with advantages thereof, will be apparent in view of the following detailed description.

## BRIEF DESCRIPTION OF THE DRAWINGS

The following detailed description will be more fully understood in view of the drawing in which:

Fig. 1 is an X-ray diffraction (XRD) spectra of the gradient coating of Samples 1 and 2 described in Example 2, showing the presence of anatase on the surfaces, wherein, in Sample 1, the anatase grains have a size of about 15 nm according to Scherrer's equation (see Cullity, Elements of X-ray Diffraction, Addison-Wesley, Reading, MA (1978)), and Sample 2 exhibits a higher crystallinity than Sample 1, with the anatase grain size increasing as a result of the heat treatment to about 35 nm.

Fig. 2 shows XRD spectra for the coatings described in Example 8 after the physical vapor deposition (PVD) process. The location of peaks stemming from anatase and rutile structures as well as from pure titanium are indicated.

Fig. 3a is a scanning transmission electron microscopy (STEM) image showing the titanium oxide layer in Example 8 in cross-section, with the coating features marked therein.

Fig. 3b shows the corresponding line profiles of titanium and oxygen over the marked line from 0 to 150 nm as obtained by energy dispersive X-ray spectroscopy (EDS).

Fig. 4 shows X-ray photoelectron spectroscopy (XPS) analysis of the HA coatings described in Example 8, displaying the elemental content of the coating surface.

Fig. 5 shows a scanning electron microscopy (SEM) image of the HA coating biomimetically grown on anatase $TiO_2$ for four days at 60°C in Example 8. An XRD spectrum of the surface is shown in the inset. Location of peaks stemming from crystalline HA are indicated.

Fig. 6 shows images of inhibition zones of bacteria growth in *Staphylococcus aureus*-containing agar close to HA covered plates loaded with (a) Tobramycin, (b) Amoxicillin, and (c) Cephalothin at the loading times indicated on the left vertical axis, and at (d) shows the turbid agar solution close to the unloaded HA reference, all as described in Example 8. Before immersion in bacterial agar, the antibiotics loaded plates were stored in PBS for the release times indicated on the horizontal axis.

Figs. 7a and 7b show release curves describing the amount of Cephalothin and Amoxicillin, respectively, released in room tempered de-ionized water per surface area ($cm^2$) HA coated plates that had been loaded with the antibiotics by soaking during the displayed loading times, as described in Example 8.

Fig. 8 shows release curves describing the amount of Cephalothin and Amoxicillin released in room tempered PBS per surface area ($cm^2$) HA coated plates that had been loaded with the antibiotics by soaking during the displayed loading times, as described in Example 8.

Fig. 9 shows atomic concentration of elements of sample (S) surfaces described in Example 9 prepared by soaking in a pamidronate containing buffer for 15 and 60 minutes, and an HA Reference material (HA Ref), respectively, as indicated. The values are average values after measurements made on 2 samples and the error bars denote the absolute deviation of these experiments

Fig. 10 shows an SEM image of hydroxyapatite crystals on Sample S60 (the sample soaked for 60 minutes) described in Example 9.

Fig. 11 shows an X-ray diffraction spectrum of Sample S60 described in Example 9.

Fig. 12 shows the conductance of a solution surrounding Sample S60 and a HA-Ref sample in the alternating ionic current (AIC) experiment described in Example 9.

Fig. 13 shows a schematic diagram of the in vitro set-up used in the cell proliferation experiment described in Example 13.

Fig. 14 shows the cell differentiation (in units of absorbance at 405 nm) on a native amorphous $TiO_2$ covered substrate (Sample A), a crystalline $TiO_2$ covered sample (Sample B), and a BMP-2 containing HA covered sample (Sample D) after 24 and 48 hours, as described in Example 14.

Fig. 15 shows SEM images of Samples A, B, C and D with cells on the surface as further described in Example 15.

The embodiments set forth in the drawing are illustrative in nature and are not intended to be limiting of the invention defined by the claims. Moreover, individual features of the drawing and the invention will be more fully apparent and understood in view of the detailed description.

DETAILED DESCRIPTION

The present invention is directed to crystalline composite materials, surgical implants, kits, methods of manufacturing composite materials, and methods of preparing a surgical implant composite material for implantation in an individual. The following detailed description shows various modes of carrying out the invention. The description is not to be taken in a limiting sense, but is made for the purpose of illustrating the general principles of the invention.

PCT/IB2007/054505 (hereafter referred to as PCT '505), describes composite materials, for example, surgical implant composite materials, kits including such materials, and methods of manufacturing such materials. PCT '505 discloses one embodiment of a surgical implant composite material comprising a surgical implant substrate and a thin film coating deposited on the substrate, the thin film coating comprising $TiO_{2-x}M_y$, wherein M is one or more elements which does not adversely effect adherence of the coating to the substrate, y is the sum of the mols of all M elements, $0 \leq x < 2$ and $0 \leq y \leq 1$, and wherein an outermost portion of the thin film coating is crystalline. Another embodiment of PCT '505 describes a method of forming a surgical implant composite material, which method comprises depositing a thin film coating on a surgical implant substrate, the thin film coating comprising $TiO_{2-x}M_y$, wherein M is one or more elements which does not adversely effect adherence of the coating to the substrate, y is the sum of the mols of all M elements, $0 \leq x < 2$ and $0 \leq y \leq 1$, and wherein an outermost portion of the thin film coating is crystalline. In one of the examples in PCT '505, such coatings were deposited on Ti substrates using reactive magnetron sputtering of titanium in argon and an oxygen partial pressure, with a substrate heating of 350°C. This procedure produced coatings that were crystalline according to X-ray diffraction and on which HA was foamed, and thus proven to be bioactive, when soaked in simulated body fluid (SBF) or in phosphate buffered saline (PBS). Crystalline Ti oxide containing coatings deposited on substrates heated to temperatures below about 400-500°C by chemical or physical vapor deposition techniques are usually not monocrystalline but instead contain crystalline grains.

The composite materials according to the present invention comprise a substrate and a thin film coating deposited on the substrate. The thin film coating exhibits high adhesion to the substrate and therefore is advantageous for use in various applications. The thin film coating comprises $TiO_{2-x}M_y$, wherein M is one or more elements which does not adversely effect the adherence of the coating to the substrate, y is the sum of the mols of all of M elements, $0 \leq x < 2$ and $0 \leq y \leq 1$. An outer most portion of the thin film coating is crystalline, with crystalline grains larger than 1 nm. The phase composition of the outer most portion is mainly, i.e., greater than 50 mol percent, crystalline, i.e., anatase, rutile or combinations thereof. Here and throughout the present specification, reference to "crystalline" in the context of the invention shall be taken to include all phases or mixtures of phases that produce crystalline peaks in an X-ray diffraction measurement. Crystalline structures thus include, but are not limited to, structures that are monocrystalline, polycrystalline, microcrystalline, nanocrystalline, and combinations thereof, as well as structures consisting of crystalline grains, for example, grains larger than 1 nm, embedded in an amorphous matrix.

More specifically, the phase composition of the outer most portion is greater than 60 mol percent crystalline, and, in more specific embodiments, is greater than 70 mol percent, 80 mole percent, 90 mol percent, 95 mol percent, and 99 mol percent, crystalline, respectively. In one embodiment, the crystalline grains (crystallites) are larger than 5 nm. In a more specific embodiments, the crystalline grains are larger than 10 nm, larger than 15 nm, larger than 20 nm and larger than 30 nm, respectively. In yet further embodiments, the crystalline grains are larger than 35 nm, larger than 40 nm, larger than 45 nm and larger than 50 nm, respectively. Throughout the present disclosure and claims, crystalline grain size is measured according to Scherrer's equation on X-ray diffractograms. Scherrer's equation is used frequently in X-ray analysis of materials. It relates the peak breadth of a specific phase of a material to the mean crystallite size of the material as follows:

$$L = (K \cdot \lambda) / (\beta \cdot \cos\theta)$$

where L is the crystallite length, P is the width of the peak at half maximum intensity of a specific phase in radians, K is a constant that varies with the method of taking the breadth ($0.89 < K < 1$), $\lambda$ is the wavelength of incident x-rays, and $\theta$ is the center angle of the peak. See, "The Scherrer Formula for X-Ray Particle Size Determination," Phys. Rev. (American Physical Society), 56(10):978-982 (November 1939), and Elements of X-ray Diffraction, Third Edition, Cullity et al (2001).

The composite materials of the invention therefore exhibit increased crystallinity as compared with various prior art materials. Throughout the present disclosure and claims, increased crystallinity refers to increased grain size and/or increased number of crystalline grains, thus producing narrower and better defined peaks in an X-ray diffractogram. This increased crystallinity is desirable as it contributes to increase the bioactivity of the titanium oxide thin films, for example, on medical implants. In one embodiment, increased crystallinity may be provided in titanium dioxide-containing thin films made by chemical vapor deposition and physical vapor deposition techniques, including but not limited to sputtering and evaporation, by annealing the thin films at temperatures sufficient to increase the crystallinity. Such temperatures may be as low as 300-400°C, and therefore sufficiently low to avoid compromising the mechanical stability of the underlying substrate. However, other techniques may be apparent in view of the present description and are within the scope of the invention.

In one embodiment of the invention, the substrate comprises a surgical implant. Surgical implants are widely used in, e.g., otology, orthopedics, dentistry treatments of bone or soft tissue defects, and as cardio vascular implants, and the surgical implant substrate for use in the present invention may take any implant form known in the art. The substrate, for example, the surgical implant substrate, may be formed of any suitable material, including, but not limited to, metals, e.g. titanium, titanium alloys, stainless steel, and Co-Cr alloys, tantalum, ceramics, e.g., zirconium dioxide and aluminum oxide, and polymers. Thus, the composite materials according to the invention are versatile and may be used in various applications, including, but not limited to, surgical implants, owing to the variety of substrate compositions which may be employed therein. In the following description, reference is made to surgical implant substrates. It will be appreciated however that the substrate may be suitable for use in other applications and therefore that the present description is not limited to composite materials employing surgical implant substrates.

The surgical implant substrate is coated by depositing a thin film coating on the substrate surface. The coating comprises titanium and oxygen, and optionally, M, wherein M is one or more elements that do not adversely affect adherence of the coating to the substrate, i.e., that together with Ti provides a material which has good adherence to the implant surface. For example, M may include, but is not limited to, Ag, I, Si, Ca, Zr, Hf, P, C, N, or any combination thereof. In a specific embodiment, M is nitrogen or carbon or a combination of the two. The stoichiometric ratio between Ti and M, can vary within the ranges of $TiO_{2-x}M_y$, wherein y is the sum of the mols of all M elements, $0 \leq y \leq 1$. In one embodiment, Ti is the dominating part of the combination.

The thin film coating may be formed to any desired thickness. The thickness of the thin film coating may be selected based on the intended composite material application. In one embodiment, the thin film coating has a thickness less than about 100 $\mu$m, or more specifically, less than about 50 $\mu$m. In a more specific embodiment, the thin film coating has a thickness less than about 30 $\mu$m, or more specifically, less than about 10 $\mu$m. In a further embodiment, the thin film coating has a thickness less than about 1 $\mu$m, or more specifically, less than about 500 nm.

In one embodiment, the thin film coating has a gradient composition through at least a portion of the thin film coating thickness. In a specific embodiment, the thin film coating is substantially free of oxygen at the substrate surface. Thus, in one further embodiment, the thin film coating is substantially pure Ti metal at the substrate surface. Further, in a specific embodiment, the gradient composition increases in oxygen content in a direction extending from the substrate toward the thin film coating surface. The gradient composition may vary in any manner, for example, monotonically or in a stepwise manner. Further, the gradient composition may vary monotonically in a linear, parabolic, or exponential manner.

In a further embodiment, the thin film coating comprises $TiO_2$ as the outermost part of the coating, and the thin film coating has a gradient composition through at least a portion of the thin film coating thickness. In a specific embodiment, the coating adjacent the substrate surface is substantially Ti and the coating composition contains an increasing oxygen concentration towards the coating surface, finally reaching a composition consisting substantially of $TiO_2$ as the outermost part. However, indicated amounts of one or more M elements are acceptable as long as they do not interfere with the substrate adhesion of the coating and are typically included to provide improved substrate adhesion and/or coating functionality such as bioactivity, mechanical stability, surface configuration, i.e., porosity, surface charge, or the like.

When the thin film coating includes a gradient composition, the gradient composition may comprise from 99% to 0.01% of the thin film coating thickness. In a more specific embodiment, the gradient composition may comprise less than about 90% of the thin film coating thickness. In a further embodiment, the gradient composition comprises at least about 10% of the thin film coating thickness. In a specific embodiment, the gradient composition has a thickness of greater than about 7 nm, more specifically greater than about 15 nm, and even more specifically greater than about 40 nm, and/or a thickness less than about 30 $\mu$m, more specifically less than about 1 $\mu$m, and even more specifically less than about 200 nm. In a further specific embodiment, the gradient composition has a thickness of from about 40 nm to 200 nm.

The thin film coating is provided on the substrate by deposition, and the crystallinity may be provided by controlling the deposition method, e.g., by using chemical vapor deposition (CVD), e.g., laser chemical vapor deposition or low temperature chemical vapor deposition, physical vapor deposition (PVD), e.g., sputtering and evaporation techniques, atomic layer deposition (ALD), or ion beam assisted deposition. These coating techniques allow the formation of coating surfaces with a surface roughness of less than about 20 micrometers to be uniformly coated.

In one embodiment, the thin film coating is deposited on the substrate using reactive magnetron sputtering of titanium in argon and an oxygen partial pressure, with substrate heating. Sputtering techniques in general are disclosed by Safi, "Recent aspects concerning DC reactive magnetron sputtering of thin films: a review," Surface and Costings Technology, 127:203-219

(2000). Techniques to deposit thin titanium oxide films specifically are disclosed by Guerin et al, "Reactive-sputtering of titanium oxide thin films," Journal of Vacuum Science & Technology A, 15(3):712-715 (1997); Baroch et al, "Reactive magnetron sputtering of TiOx films," Surface & Coatings Techrrology, 193:107-111 (2005); Barnes et al, "The mechanism of TiO2 deposition by direct current magnetron reactive sputtering," Thin Solid Films, 446:29-36 (2004); and Barnes et al, "The mechanism of low temperature deposition of crystalline anatase by DC magnetron sputtering," Sulface & Costings Technology, 190:321-330 (2005).

In another embodiment of the invention, the thin film coating is deposited on a substrate using evaporation of Ti under oxygen pressure to form a Ti oxide-containing surface.

In one specific embodiment of the invention, the method of forming the thin film comprises a post deposition heat treatment or annealing step, conducted at a temperature sufficient to increase the crystallinity of the thin film coating. The increased crystallinity of the thin film coating contributes to improving the bioactivity of the coating. Improved bioactivity, in turn, increase the chances of successful incorporation and functioning of an implant device in the body. Preferably, the heat treatment is conducted at a temperature low enough to avoid compromising the mechanical stability of the substrate. For a thin film formed on a Ti-containing substrate, the heat treatment temperature is, in one embodiment, below 500°C, more specifically below 450°C, even more specifically below 400°C. For a thin film formed on a substrate mainly containing Co-Cr, the heat treatment temperature is, in one embodiment, below 900°C, more specifically below 600°C, even more specifically below 500°C. For a thin film formed on a substrate mainly containing stainless steel, the heat treatment temperature is, in one embodiment, below 600°C, more specifically below 500°C, even more specifically below 400°C

The heat treatment is typically performed for less than 24 hours, although longer heat treatments may be conducted, if desired. In one embodiment, the heat treatment is conducted for less than 10 hours, and in another embodiment, the heat treatment is conducted for less than 2 hours. The heat treatment can be done in the presence of any gas that does not adversely affect the functionality of the coating. Examples of such gases include, but are not limited to, air, oxygen, nitrogen, argon, helium, and krypton, and any mixture thereof. In view of the present specification teachings, one of ordinary skill in the art will be able to appreciate the appropriate times and temperatures suitable for other types of substrates.

In a specific embodiment, to increase the coating adhesion, the substrate is first cleaned using conventional cleaning procedures before conducting the deposition process for forming the thin film coating. Further, the substrate may also be sputter etched before depositing the thin film coating, for example, in order to remove native oxide on a metal implant. Typically, such sputter etching is not employed in the manufacture of composite materials using ceramic and/or polymeric implants substrates.

If desired, the coating may be porous, and in one embodiment, may be nanoporous, having pores of a size in the range of about 0.1-100 nm. Porosity of the coating may be controlled via the deposition process for example, by temperature and, when sputtering is employed, the partial pressures of argon and oxygen, mainly that of the argon pressure.

In a specific embodiment, a convenient method for depositing the thin film coating is by a sputtering or evaporation technique, wherein the titanium dioxide phase of the deposited coating is controlled via the temperature of the substrate, typically using a temperature range of from greater than room temperature to below 400 °C, in combination with the oxygen flux in the coating chamber. The partial pressure of oxygen in the coating chamber may, for example, be in the range of from about 0.01 to 5 Pa, preferably from about 0.1 to 1 Pa. The argon pressure range may be greater than 0.1 mTorr to 30 mTorr. The use of low temperature and high argon pressure results in a more porous film than does the use of a high temperature and low argon pressure. Accordingly, porosity can be controlled by adjusting the temperature and pressure parameters. In addition, the depth of the pores can be controlled by changing the reaction conditions at an appropriate time of the process. One of ordinary skill in the art will appreciate that the foregoing description is one of many deposition techniques known in the art that can be used in forming a thin film coating according to the present invention.

In a specific method of depositing the thin film coating on a surgical implant substrate, the coating adhesion may be increased by first depositing a Ti layer, or a layer containing Ti and N or any other suitable element M which in combination with Ti gives good adhesion on the substrate. In a more specific embodiment, pure Ti metal is first deposited on the surgical implant substrate. The partial pressure of oxygen may then be increased gradually to obtain a gradient coating going from the pure Ti metal, or $TiM_y$ (y being equal to or less than 1) at the interface between the implant surface and the film coating to the desired crystalline titanium oxide phase at the thin film coating surface. In another embodiment, the method comprises co-sputtering an element M, for example carbon, and Ti under nitrogen and/or oxygen pressure to form a $Ti(O_{2-x}, C_{y1}, N_{y2})$ film, where $0 \leq x < 2$, $y_1 + y_2 = y$, and $0 \leq y \leq 1$. The partial pressure of nitrogen and oxygen and the sputter rate of carbon can be controlled to form any gradient structure in the film. In yet another embodiment, the thin film coating may be formed by evaporating Ti and sputtering C under nitrogen and/or oxygen pressure to form a $Ti(O_{2-x}, C_{y1}, N_{y2})$ film, where x, y1 and y2 are as defined above. The partial pressure of nitrogen and oxygen and the sputter rate of carbon can be controlled to form any gradient structure in the film within the composition ranges.

In a specific embodiment of the invention, the thin film titanium oxide coating is deposited with porous properties and therefore is suitable for loading with a releasable agent having desired functional properties, such as, for example, an active pharmaceutical ingredient (drug), bio molecule, or ion, or the like, or a combination thereof. A surgical implant composite material including a releasable agent loaded in the thin film coating is advantageous for targeted and/or controlled release of the agent *in vivo*. The porous titanium oxide coating on a surgical implant substrate can be loaded with a releasable agent such as a drug, ion or bio molecule, or combinations thereof, via any loading technique known in the art. Examples of such techniques include, but are not limited to, soaking or vacuum impregnating the coating with a diluted suspension of the releasable agent for later delivery in vivo from the thin film coating. Other examples include absorption loading, solution loading, evaporation loading (e.g. rotary evaporation loading), solvent loading, air suspension coating techniques, precipitation techniques, spray-coagulation methods, or combinations thereof. Specific examples of releasable agents suitable for use in this embodiment include, but are not limited to, antibiotics, e.g., gentamicin, such as gentamicin sulfate, and other aminoglycosides such as amikacin, kanamycin, neomycin, netilinicin, paromomycin, streptomycin, tobramycin, and apramycin, bone morphogenesis proteins, peptides, bisphosphonates, opioids, opiates, vitamins anti-cancer drugs, iodine, Ag, combinations thereof, and the like. One of ordinary skill in the art will appreciate that the porosity of the thin film coating may be configured in order to provide a desired rate of release of the releasable agent therefrom.

In a further embodiment, the composite material comprising a surgical implant substrate and the thin film coating may further include a biomimetic coating provided on the surface of the thin film coating. Biomimetic coatings are known in the art and any suitable biomimetic may be employed herein. In one embodiment, the biomimetic coating comprises a calcium phosphate, for example an apatite, for example, hydroxyapatite (HA), $Ca_{10}(PO_4)_6(OH)_2$, brushite, $CaHPO_4 \cdot 2H_2O$, or the like. The pH of the solution may be varied to control the composition which is formed, with brushite being formed at lower pH values. The thickness of the biomimetic coating may be selected based on the application of the composite material as desired. In one embodiment, wherein the composite material includes a surgical implant substrate, the biomimetic coating has thickness less than about 200 $\mu$m, and more specifically has a thickness less than about 50 $\mu$m. In a further embodiment, the biomimetic coating has a thickness less than about 5 $\mu$m. The composite material may be provided with the biomimetic coating by any suitable coating technique known in the art. For example, HA can be synthesized and deposited on implants by several coating procedures such as plasma spraying, pulsed laser deposition, electron-beam deposition, and by biomimetic precipitation. While native amorphous $TiO_2$ has very poor ability to form HA through biomimetic precipitation from a solution on its surface, the anatase and rutile phases of $TiO_2$ allow HA to spontaneously crystallize when the $TiO_2$ is soaked in simulated body fluid.

In one embodiment, the composite material comprising a surgical implant substrate and the thin film coating as described is immersed in a calcium phosphate buffer solution for biomimetic deposition of HA or brushite, or a similar material. The deposition rate and structure is controlled via solution temperature, composition and solution strength. Brushite is deposited from buffer solutions with a pH of below 4.2 and HA or deficient HA is deposited from buffer solutions with a pH above 4.2. In a specific embodiment, HA is deposited via simulated body fluid or phosphate buffer saline. Preferably, the solution temperature is below 95°C, more specifically below 70 °C, and more specifically at about 37°C. For example, the titanium dioxide thin film coated implant substrate is immersed for a time period of up to several months in the solution, preferably up to 7 days, at about 37°C.

Optionally, the biomimetic coating can be loaded with a releasable agent having desired functional properties as described above, via any loading technique known in the art, as discussed above. Such loading of the biomimetic coating is advantageous for targeted and/or controlled release of the agent *in vivo*. In one embodiment, the releasable agent can be loaded in parallel with and/or subsequent to the formation of the biomimetic coating. In specific embodiments, for example, a surgical implant substrate having a sputter deposited thin film coating of nanocrystalline rutile $TiO_2$, with or without a gradient structure, is immersed in a simulated body fluid for up to seven days at a temperature of 60°C to form a biomimetic coating of hydroxyapatite (HA) thereon. An active substance, for example, an antibiotic such as gentamicin, is then soaked into the HA biomimetic layer formed on the surface of the thin film. In another example, a surgical implant substrate having a sputter deposited thin film coating of nanocrystalline rutile $TiO_2$, with or without a gradient structure is stored in simulated body fluid at 37°C for 48 hours to allow HA to form nuclei on the $TiO_2$ thin film coating. After that, an active substance, for example, bisphosphonate, is mixed with the simulated body fluid and allowed to co-precipitate and/or co-crystallize with the HA. In yet another example, a thin film of HA is allowed to form on the $TiO_2$ thin film coating, after which the composite material is alternately soaked in two separate solutions, one solution containing the active substance and the other solution containing simulated body fluid until the desired thickness of the active substance-containing HA biomimetic layer is formed.

In a specific embodiment, a composite material according to the invention is provided with an HA biomimetic coating and the coating is functionalized with an active agent, for example, based on the needs of the individual receiving the implant. While the prior art describes many attempts to design an implant material with a functionalized surface coating for local drug administration, fast-functionalization, i.e., functionalizing an implant surface at the site of surgery in order to provide local treatment in vivo that is optimized for the patient's need, has been difficult. Embodiments of the present invention overcome this deficiency in the art. For example, a composite material including an HA biomimetic coating having an antibiotic loaded thereon can be used to provide implants with improved biological stability, i.e., by inhibiting bacterial adhesion onto the implant, preventing biofilm adhesion, and/or direct killing of bacteria. The present methods allow fast-loading of biomimetic HA coatings with antibiotics in order to create the platform required for a multifunctional implant device where the implant and the drug are separate before implantation. The present methods can be used to create a device which 1) offers both sustained local anti-bacterial treatment at the site of the implant, thus, avoiding systemic administration of antibiotics, 2) avoids problems with safe drug handling and storage, and 3) allows a surgeon at the site of surgery to choose which drug to incorporate in the implant based on the patient's need. The present method can also be used for other active pharmaceutical ingredients and biomolecules that preferably release locally in vivo. Additionally, combinations of two or more pharmaceutically active ingredients can be loaded into the implant coatings if desired. As will be apparent, active substance release at the site of the implant can be used as a supplement to other forms of active substance administration as well.

The releasable agent may be released from the biomimetic coating while maintaining the integrity of the biomimetic coating on the $TiO_2$ thin film. Alternatively, the releasable agent may be released with the biomimetic coating, i.e., as the biomimetic layer itself is released from the $TiO_2$ thin film over an extended period of time. It will also be appreciated that a releasable agent may be released by a combination of such mechanisms. The type of release may be tailored depending on the particular agent, the biomimetic coating formation technique, and/or the loading technique.

In yet another embodiment, the loaded implant coating is rinsed or soaked in a solution before implantation in order to remove excess releasable agents present on the implant surface that may cause an unpredictable or irregular release profile, for example a "burst," during the first minutes after implantation. Non-limiting examples of suitable rinsing or soaking solutions are simulated body fluids, for example, PBS, de-ionized or distilled water, or ethanol. In one embodiment, the rinsing or soaking procedure is carried out for only a short time, for example, for 1 second to 120 minutes, more specifically, for 10 seconds to 15 minutes, or even more specifically for 15 seconds to 10 minutes, in order to remove excess agent without diminishing the availability of agent for controlled release.

On the other hand, in an additional embodiment, the loaded implant coating is not rinsed or soaked in a solution before implantation to ensure an initial "burst" or spike release of the agent occurs during the first minutes after implantation, followed by a controlled or sustained release as described herein.

Optionally, the loaded coating, which may be either the thin film coating or the biomimetic coating, may in turn be coated with a resorbable polymer for extended release of the releasable agent. The resorbable polymer can be applied via any suitable technique, for example by solution, melting or spraying onto the coated substrate, and drying or solidifying. The thickness of the polymer layer is suitably below 200 $\mu$m, preferably below 50 $\mu$m. Examples of such polymers include, but are not limited to, polylactic acids, propylene fumarate and chitosan. Optionally, cyclodextrin can be used to obtain slow release of the releasable agent as well.

In another aspect of the invention, a kit is provided comprising a surgical implant composite material comprising a surgical implant substrate and a thin film coating of titanium oxide as described herein, optionally including a biomimetic coating, together with one or more solutions of a releasable agent having a desired functional property, i.e., an active pharmaceutical ingredient, an ion or a biomolecule. The solution is operable to load the releasable agent onto the surgical implant composite material upon contact of the solution with the surgical implant composite material. Thus, prior to implantation, a specific composite material may be combined with a selected releasable agent based on the needs of the specific patient for which the implant is intended. Accordingly, in another embodiment, the invention is directed to a method of preparing a surgical implant composite material for implantation in an individual. The method comprises selecting a releasable agent to be loaded on the surgical implant composite material based on a condition of the individual, and contacting the surgical implant composite material with a solution of the releasable agent to load the releasable agent onto the surgical implant composite material. Such methods allow a surgeon to select a particular active agent, for example, a particular antibiotic, or the like, based on the characteristics and needs of the individual who is to receive the implant.

Various embodiments of the composite materials and methods of the invention are described in the following examples.

Example 1

This example demonstrates composite materials according to the invention. A surgical implant substrate having a sputter deposited thin film coating of nanocrystalline rutile $TiO_2$, without a gradient structure, is immersed in a simulated body fluid for seven days at a temperature of 60°C to form a biomimetic coating of hydroxyapatite (HA) thereon. An active substance, for example, gentamicin, is then soaked into the HA biomimetic layer formed on the surface. In another example, the substrate with the thin film coating is stored in simulated body fluid at 37°C to allow HA to form nuclei on the $TiO_2$ thin film coating. An active substance, for example, bisphosphonate, is then mixed with the simulated body fluid and allowed to co-precipitate and/or co-crystallize with the HA. In yet another example, a thin film of HA is allowed to form on the $TiO_2$ thin film coating, after which the composite material is alternately soaked in two separate solutions, one solution containing the active substance and the other solution containing simulated body fluid until the desired thickness of the HA-containing biomimetic layer is formed.

Example 2

A series of experiments were performed to deposit titanium oxide coatings on metallic substrates. Specifically, graded titanium dioxide thin films were prepared in a reactive DC magnetron sputtering unit (Balzers 640R). The sample holder was rotated and a pure titanium target (99.9%) was used for depositing a thin film layer. Pure argon (99.997%) and oxygen (99.997%) were used for the reactive sputtering. The magnetron effect and oxygen partial pressure were chosen to 1.5 kW and 1.5 $10^{-3}$ mbar, respectively.

In a first experiment (Experiment 1), a first set of samples were deposited by first depositing a layer of pure titanium of 50 nm thickness. On the surface of this pure titanium layer, a second layer of 50 nm was formed with the oxygen flow gradually increasing from near zero to a constant value to give an oxygen content gradient in the resulting Ti oxide layer. When the oxygen flow was high enough to produce $TiO_2$, the flow was held constant at this flow to form a 100 nm thick $TiO_2$ layer. The substrate temperature during these steps was held constant at 350°C. The resulting material is referred to as Sample 1. A second experiment (Experiment 2) was conducted by repeating the procedure in Experiment 1 and by heat treating the sample for 1 hour in air at 390°C post deposition. The resulting material is referred to as Sample 2. A third experiment (Experiment 3) was conducted by depositing the titanium oxide coating without substrate heating and no heat treatment post deposition. The resulting material is referred to as Sample 3. A fourth experiment (Experiment 4) was conducted by repeating the procedure in the third experiment and by heat treating the sample for 1 hour in air at 390°C post deposition. The resulting material is referred to as Sample 4.

The obtained coatings were characterized using X-ray diffraction (XRD) for phase composition (detection of crystalline phases), scanning electron microscopy (SEM) for studying the film thickness in cross-section (LEO 440), and X-ray photoelectron spectroscopy (XPS) for detecting the gradient structure. The coating adhesion was measured using Rockwell C indentation.

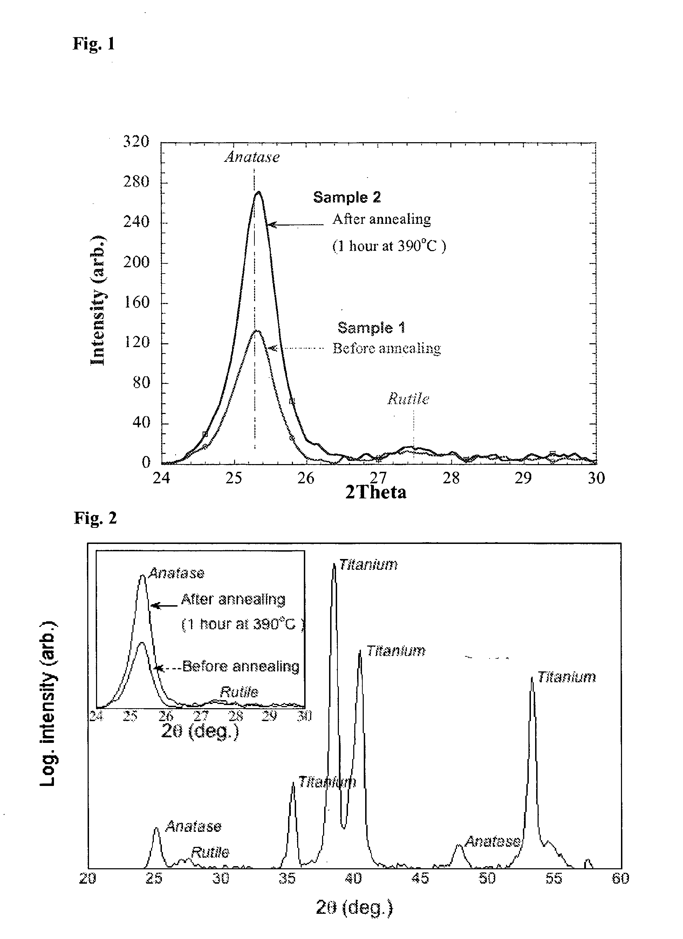

The outermost region of the coatings of Samples 1 and 2, deposited at 350°C, were nanocrystalline. The crystallinity of Sample 2, produced with the post deposition heat treatment, was higher than that of Sample 1. As shown in Fig. 1, Sample 1 contained anatase phase $TiO_2$ with a grain size according to Scherrer's equation of about 15 nm while Sample 2 contained mainly anatase phase $TiO_2$ with a grain size according to Scherrer's equation of about 35 nm.

Analyzing Samples 3 and 4 using XRD showed that the coating deposited with no substrate heating and without post deposition treatment (Sample 3) was amorphous and that the sample deposited without substrate heating but with post deposition heating (Sample 4) was crystalline.

XPS analysis of the gradient coatings showed that the gradient zones in the coatings were about 50 nm as is evident from XPS depth profiles. Substrate adhesion testing using Rockwell C indentation showed that the adhesion was the highest for the gradient coatings deposited using substrate heating, Samples 1 and 2. The heat treatment of Sample 2 did not seem to affect the adherence as no difference could be found between Samples 1 and 2 in the adhesion testing.

These experiments show that the titanium oxide-containing coatings having a gradient in the oxygen content and deposited at 350°C (Samples 1 and 2) were nanocrystalline and had a high adhesion. They also show that samples that are sputtered with substrate heating and thereafter heat treated (Sample 2) have higher crystallinity than those that are not heat treated (Sample 1). They further show that samples that are amorphous after sputter deposition at room temperature (Sample 3) can be made crystalline by only moderate heat treatment (Sample 4).

Example 3

Samples 1-4 from Example 2 were tested for their in vitro bioactivity following the method of Kokubo et al, "How useful is SBF in predicting in vivo bone bioactivity?," Biomaterials, 27:2907-2915 (2006). Soaking bone bioactive materials in simulated body fluid (SBF) or phosphate buffered saline (PBS) results in the formation of a biomimetic HA coating on the surface. Initially, the titanium oxide coated implants from Example 2 were ultrasonically cleaned, at a resonance of 20 kHz, for 5 min each in a bath of acetone, a bath of ethanol and finally a bath of deionized water. The coated Ti implants were then immediately immersed in 40 ml of 37°C preheated phosphate buffered saline (PBS, Dulbecco's Phosphate Buffered Saline, Sigma-Aldrich Company Ltd.) in falcon tubes. The presence of HA was detected using thin film X-ray diffraction (XRD) and scanning electron microscopy (SEM).

After 7 days in the PBS solution a uniform, porous biomimetic hydroxyapatite layer was formed on the nanocrystalline titanium dioxide surface of Samples 1, 2 and 4, indicating the $TiO_2$ surface was bioactive. The coating of Sample 3 deposited at ambient temperature did not show a biomimetic hydroxyapatite layer on the surface and the coating was not bioactive.

Example 4

The soaking experiment was repeated for Samples 1-4 from Example 2 using various soaking times. It was found that a uniform, porous biomimetic hydroxyapatite layer was formed on the nanocrystalline titanium dioxide surface of Sample 2 faster than on Sample 1, indicating that a larger crystallinity produced by the heat treatment increased the bioactivity of the coatings.

Example 5

A series of drug loading experiments were performed on the HA coated crystalline titanium oxide coatings in the samples from Example 3. Specifically, the drug loading employed the antibiotic drug gentamicin sulfate (GS), incorporated in the hydroxyapatite layer (HA). All tests were performed at 37°C in falcon tubes. In a first series of experiments, two soaking procedures were tested:

  • Soaking the HA coatings from Example 3 in a water-GS solution for 3 days (Experiment 6).
  • Soaking the HA coatings from Example 3 in a PBS-GS solution for 7 days (Experiment 7). This allows a co-precipitation and/or co-crystallization of both GS and HA.

In a second series of experiments, ethanol was added to the solution (10 wt%) to reduce the solubility of GS. These experiments were denoted Experiments 8 and 9.
The release of GS from the implants was studied using *Staphylococcus aureus*, *Staphylococcus epidermidis*, and *Pseudomonas aeruginosa* bacteria. All strains were grown overnight on Muller-Hinton (Difco) agar plates at 37°C. Samples 6-9, resulting from Experiments 6-9, respectively, were put into the agar plates and the diameter of the bacteria death surrounding the samples was measured as a function of time.
All samples showed antibacterial effect for more than 24 hours with Samples 8 and 9 showing a wider effected diameter than Samples 6 and 7. Samples 8 and 9 also had a longer release period than Samples 6 and 7. These experiments showed a slow drug release from the HA/crystalline coatings described in Examples 2 and 3.

Example 6

In this example, the surface of a surgical implant substrate was sputter coated with a thin layer of pure Ti (20 nm) at a substrate temperature of 360 °C. On the surface of this layer, a titanium oxide layer with oxygen content increasing from 0 to 2 was sputter deposited. The thickness of the resulting gradient layer was 60 nm. As an outermost bioactive coating, a layer of nanocrystalline $TiO_2$ was sputtered (120 nm). The coating was heat treated at 350°C for 75 minutes in air, post deposition. The coated implant surface was soaked in PBS for 4 days at about 37°C to form a biomimetic HA layer. The resulting material was packed, sealed and sterilized using gamma radiation. The sterilized implant was soaked with a liquid solution containing amoxicillin for 15 minutes. The effect of the drug was studied in vitro using the standard agar plate with bacteria generally as described above. The soaked implant showed an antibacterial effect for more than 24 hours, indicating that prolonged antibacterial effect of the implant can be achieved by soaking an implant including a porous biomimetic HA layer immediately prior to implantation.

Example 7

An experiment (Experiment 10) identical to that in Experiment 1 but forming only the Ti layer and the $TiO_2$ layer thereon, without the gradient structure in between, was performed. The substrate temperature during these steps was held constant at 350°C to form Sample 10. Another experiment (Experiment 11) was conducted by repeating the procedure in Experiment 10 and by heat treating the sample for 1 hour in air at 350°C post deposition. The resulting material is referred to as Sample 11. Another experiment (Experiment 12) was conducted by repeating the procedure in Experiment 10, without the substrate heating. The resulting material is referred to as Sample 12. Another experiment (Experiment 13) was conducted by repeating the procedure in Experiment 12 and by heat treating the sample for 1 hour in air at 350°C post deposition. The resulting material is referred to as Sample 13.
Analysis of Samples 1 and 2 using XRD showed that the coating deposited with substrate heating and without post deposition treatment (Sample 10) was nanocrystalline with a grain size smaller than 10 nm and that the sample deposited with substrate heating and with post deposition (Sample 11) was crystalline and had a grain size larger than that of Sample 10. Further XRD showed that Sample 12 was amorphous while Sample 13 was nanocrystalline.
These experiments show that the titanium oxide-containing coatings without a gradient in the oxygen content and deposited at 350°C (Samples 10 and 11) were nanocrystalline. They also show that samples that are sputtered with substrate heating and thereafter heat treated (Sample 11) have higher crystallinity than those that are not heat treated (Sample 10). They also demonstrate that samples that are amorphous after sputter deposition at room temperature (Sample 12) can be made crystalline by only moderate heat treatments (Sample 13).

Samples 10-13 were tested for their in vitro bioactivity following the procedure described in Experiment 3. After 7 days in the PBS solution a uniform, porous biomimetic hydroxyapatite layer was formed on the nanocrystalline titanium dioxide surface of Sample 10, 11 and 13. The coating of Sample 12 deposited at ambient temperature did not show a biomimetic hydroxyapatite layer on the surface. The crystalline coatings were therefore bioactive and a biomimetic HA coating was formed. The amorphous coating was not bioactive.

The soaking experiment as described above was repeated for Samples 10, 11, 12 and 13 for various times in PBS. It was found that a uniform, porous biomimetic hydroxyapatite layer was formed on the nanocrystalline titanium dioxide surface of Sample 11 at a faster rate than on the surface of Sample 10, indicating that a larger crystallinity produced by the heat treatment increased the bioactivity of the coatings.

The soaking experiments of Example 5 and the kit forming experiments of Example 6 were repeated for the non gradient crystalline samples 10, 11 and 13. Sample 10 was generally found to behave like Sample 1, Sample 11 was generally found to behave like Sample 2, and Sample 13 was generally found to behave like Sample 4 in these experiments.

Example 8

This example describes and studies antibiotic incorporation in implant coatings according to the invention. As noted above, the concept of biological stability in implants focuses on three different aspects, namely, inhibition of bacterial adhesion onto the implant, prevention of biofilm formation, and direct killing of bacteria. Orthopedic as well as dental surgeons desire more flexibility with implants, including fast-functionalization of implant surfaces at the site of surgery, in order to provide local treatment in vivo that is optimized for the patient's need. HA coatings are not only bioactive but may also be functionalized to evoke a certain cell response or used as drug carriers as nano-porous HA can serve as a matrix for local administration of molecules.

This example demonstrates a method to fast-load biomimetic HA coatings with antibiotics in composite materials of the invention in order to create the platform required for a multifunctional implant device where the implant and the drug are separate before implantation. The present method creates a device which 1) offers both sustained local anti-bacterial treatment at the site of the implant, thus, avoiding systemic administration of antibiotics, 2) avoids problems with safe drug handling and storage, and 3) lets the surgeon at the site of surgery choose which drug to incorporate in the implant based on an individual patient's need. The presented method can also be used for other active pharmaceutical ingredients and biomolecules that preferably are released locally in vivo.

*Ti oxide deposition and characterization*

A coating of Ti oxide for which the oxygen content increased from the substrate towards the outermost surface was deposited on 10 x 20 mm plates of commercially pure grade 5 titanium by physical vapor deposition (PVD). The gradient coating, tailored to optimize the adhesion of the coating to the substrate, was prepared in a reactive DC magnetron-sputtering unit (Balzer UTT400). The working chamber was mounted with a turbo molecular pump TMU 521P. The chamber base pressure was ~$10^{-7}$ mbar after backing. Pure titanium (99.9%) was used as a target. Pure argon (99.997%) and oxygen (99.997%) were used for the reactive sputtering. The substrate was kept at a constant temperature of 350°C during sputtering. The oxygen profile of the film was controlled by changing the oxygen flow. An increasing oxygen flow reduces the metallic content. A crystalline outermost $TiO_2$ surface was ensured by heat treatment of the sputtered film at 390°C for one hour post deposition. The targeted coating design parameters were as follows, described from the titanium substrate of grade 5; 50 nm of pure titanium, 50 nm gradually increasing oxygen content from Ti to $TiO_2$ and a top layer of 100 nm crystalline $TiO_2$.

The thickness of the films was measured with surface profilometry, using a Tencor Alpha-step 200 instrument (USA product) and the order of crystallinity was examined using gracing incidence X-ray Diffraction (XRD, Siemens D5000 diffractometer). The Ti oxide coating was further studied by Transmission Electron Microscopy (TEM, FEI Tecnai F30 ST microscopy equipped with a field emission gun operated at 300 kV) and profiles of the elemental composition in the gradient film were achieved using energy dispersive spectroscopy (EDS) in scanning TEM mode (STEM). Electron transparent samples for the TEM analysis were produced using focused ion beam (FIB) milling in a FEI Strata DB235. A thin (~1 μm) Pt coating was deposited on the Ti oxide surface and then cut out using the ion beam (Ga3+). The sample was lifted out using an Omniprobe system and welded onto a TEM sample grid and further milled using the ion beam to electron transparency (thickness < 100 nm).

*Biomimetic coating of HA*

After PVD deposition the plates were ultrasonically cleaned in acetone, ethanol and de-ionized water (5 minutes in each) and subsequently placed in plastic tubes with conical bottoms containing 40 ml PBS (Dulbecco's PBS with calcium chloride and magnesium chloride, Sigma-Aldrich Company Ltd.) preheated to 60°C and placed in an incubator at 60°C for four days. After the immersion, the plates were gently rinsed with de-ionized water and the precipitated HA coatings were examined using Scanning Electron Microscopy (SEM, Zeiss LEO 1550), X-ray Photoelectron Spectroscopy (XPS, Physical Systems Quantum 2000 spectrometer) and gracing incidence XRD.

*Incorporation of antibiotics*

The incorporation of antibiotics was preformed by soaking the HA coated plates in 10 ml PBS containing 50 $\mu$g of either Tobramycin, Cephalotin, Amoxicillin or Gentamicin sulfate (Sigma-Aldrich Company Ltd.). The drug loading was performed at room temperature in 50 ml conical test tubes for four different soaking times between 15 minutes and 24 hours.

*Antibacterial effect in bacteria agar*

After incorporation of antibiotics, the plates were immediately transferred to new tubes filled with 40 ml of PBS preheated to 37°C in order to let the incorporated drug leak out for various time intervals before monitoring the antibacterial effect of the drug remaining in the coatings. After either 15 minutes, 60 minutes or 24 hours, the plates were removed and dried at room temperature for further analysis of the remaining antibacterial effect in the *Staphylococcus aureus* agar described below.

A Mueller-Hinton broth (Merck) containing 0.8% Agar was prepared by dissolving the powder in de-ionized water and sterilized by autoclaving at 121°C for 20 minutes. The broth was then tempered to 40°C in a water bath. *Staphylococcus aureus* strain Cowan I (NCTC 8530, National Collection of Type Cultures, London, UK) in a PBS suspension was added directly to the broth giving it a final optical density at 600 nm of 0.005 (corresponding to approximately $5 \times 10^6$ cfu/ml). The mixture was mixed to homogenously distribute the bacteria in the substrate and then transferred to Petri dishes. The dishes were filled to a depth of 10 mm (50 ml) with the bacterial broth. As the agar stiffened at room temperature, the titanium plates were stuck down in the agar at a right angle to the surface, having both sides in contact with the bacterial substrate. The Petri dishes were then incubated at 37°C for 18 hours. A HA coated reference plate without any antibiotics was also tested. The inhibition zones of bacterial growth formed around the plates were photographed by an imaging instrument (Geldoc 2000, Bio-Rad).

*Antibiotic release studied by UV spectroscopy*

The drug release rate was analyzed using UV spectroscopy (Shimadzu 1650PC, Shimadzu Corp.) by immersing plates that had been soaked in Amoxicillin and Cephalothin for 15 minutes, 1 hour and 24 hours in 8.2 ml of either water or PBS at room temperature. The release was measured (using a wavelength Of 230 nm for Amoxicillin and 239 nm for Cephalothin) in water for 1 hour to investigate the initial release and then in PBS for 22 hours to investigate the slow release after the initial burst. The liquid was circulated through a cell in which the drug concentration was detected. After the release in PBS, the HA coatings soaked for 24 hours in the antibiotic solutions were dissolved by lowering the pH of the PBS to 2 by addition of hydrochloric acid. This was done to release and measure the amount of remaining drugs still present in the coatings after 22 hours of release. To relate the antibiotics concentration released to the antibacterial effect, antimicrobial susceptibility tests (E-test, AB Biodisk, Sweden) for Amoxicillin and Cephalothin was performed on *strain S aureus* Cowan I and on *Staphylococcus epidermidis* Kx293A1 (Department of Microbiology, Swedish University of Agricultural Sciences).

*Results, Ti oxide*

The PVD process resulted in a crystalline $TiO_2$ surface, mainly of anatase phase, see Fig. 2. Diffraction peaks originated from the crystalline titanium substrate are also seen, corresponding to the different crystallographic directions of alpha-Ti. The heat treatment after the coating deposition gave a more crystalline coating structure (more defined peaks in the thin-film XRD spectra) than the as-deposited coatings.

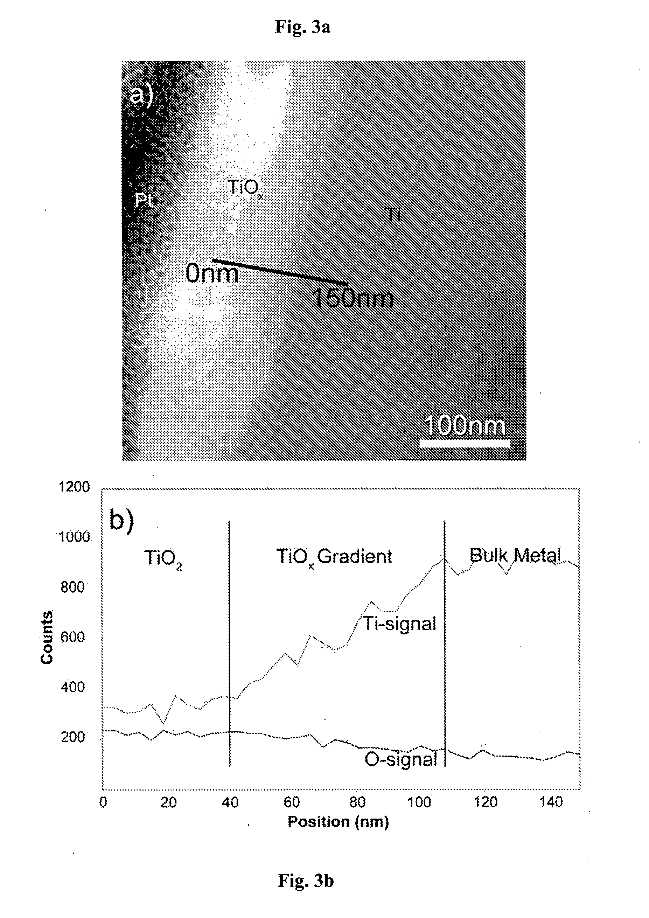

The cross-section TEM analysis showed that the coating had a thickness of about 170 nm, i.e. slightly less than the nominal thickness as measured with surface profilometry, see Fig. 3. The EDS profile (Fig. 3b) showed that the gradient extended over 70 nm. In Fig. 3a, the gradient can be seen as the contrast changes going from the surface towards the Ti-bulk.

*Results, Biomimetic Coating of HA*

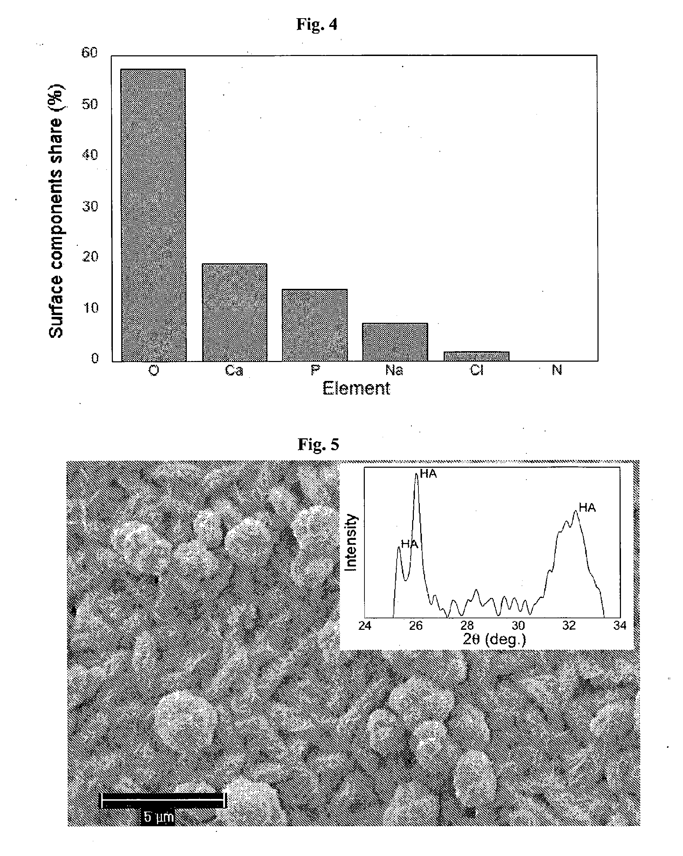

Immersion of the titanium plates in PBS resulted in biomimetic precipitation of nano-porous and calcium deficient HA on the anatase $TiO_2$ surfaces. XPS analysis showed that the Ca/P ratio of the HA coatings was 1.36 compared to stoichiometric HA with a ratio of 1.67, see Fig. 4. Further, the SEM and XRD analyses showed that a continuous layer of HA had formed all over the anatase surfaces, see Fig. 5. It has previously been shown that HA with a non-stoichiometric calcium deficient composition, like the one found here, is more favorable as a bone implant compared to stoichiometric HA because it dissolves faster in aqueous environments and induces a much more rapid bone colonization. As well, calcium deficient HA generally has a larger specific surface area than its stoichiometric counterpart, which has been found to facilitate the loading of therapeutic agents.

*Results, anti-bacterial effect in bacteria agar*

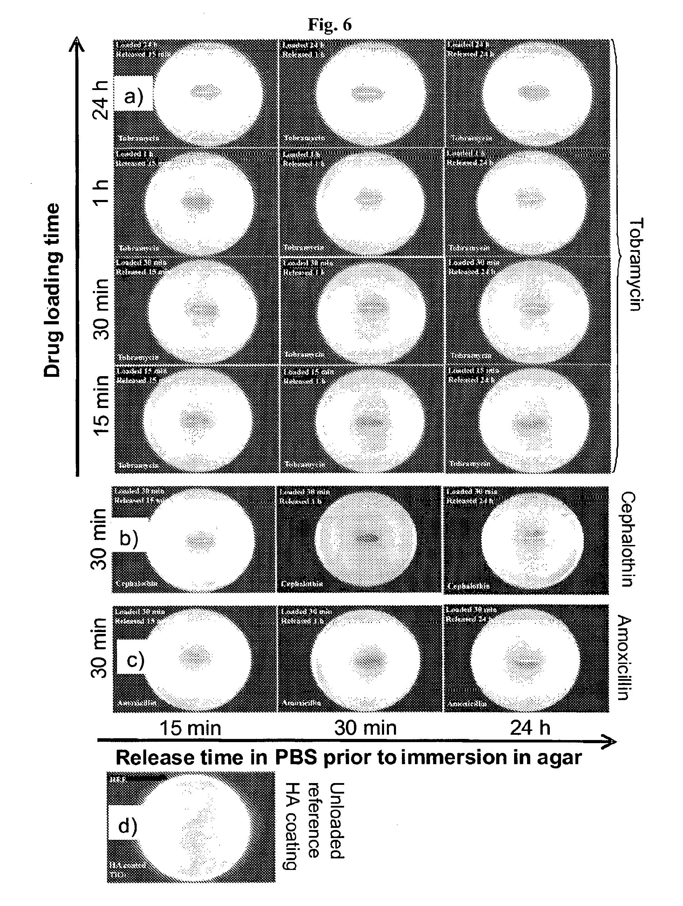

Fig. 6 shows the inhibition zones of bacteria growth adjacent the plates soaked in Tobramycin (Fig. 6a), Amoxicillin (Fig. 6b) and Cephalothin (Fig. 6c) at the indicated time periods; 15 and 30 minutes, as well as 1 and 24 hours for Tobramycin and 30 minutes for Amoxicillin and Cephalothin. Before immersion in the agar, the plates were placed in PBS for 15 minutes, 1 hour and 24 hours, respectively, in order to investigate the effect of the antibiotics possibly remaining in the coatings after the various release times. For all experiments shown in Figs. 6a-6c, as well as those performed with loading times not shown here (15 min, 1 and 24 hours for Amoxicillin and Cephalothin, 15 and 30 min as well as 1 and 24 hours for Gentamicin), the agar solution closest to the plates remained clear showing that the antibiotic effect was preserved even for the most extreme values with 15 minutes drug loading followed by release for 24 hours in PBS. The fact that the size of the clear zones closest to the plates did not show a clear size dependence on loading and/or release time further indicated that the use of loading times longer than 15 minutes does not necessarily give higher drug release rates during the first 24 hours after implantation. All of Figs. 6a-6c show the released antibiotics had an effect on both sides of the plates albeit the HA coating containing the antibiotics only covered one of the plate sides. This may be caused by a preferred diffusion direction along the sides of the plates rather than a spherical diffusion out into the agar. The turbid agar solution close to the unloaded HA surface in Fig. 6d shows that this reference coating did not have any antibacterial effect. These results experimentally demonstrate a fast-loading slow-release coating for surgical implants and creating an opportunity for fast loading of implant surfaces by a surgeon in the operating room.

*Results, antibiotics release studies by UV spectroscopy*

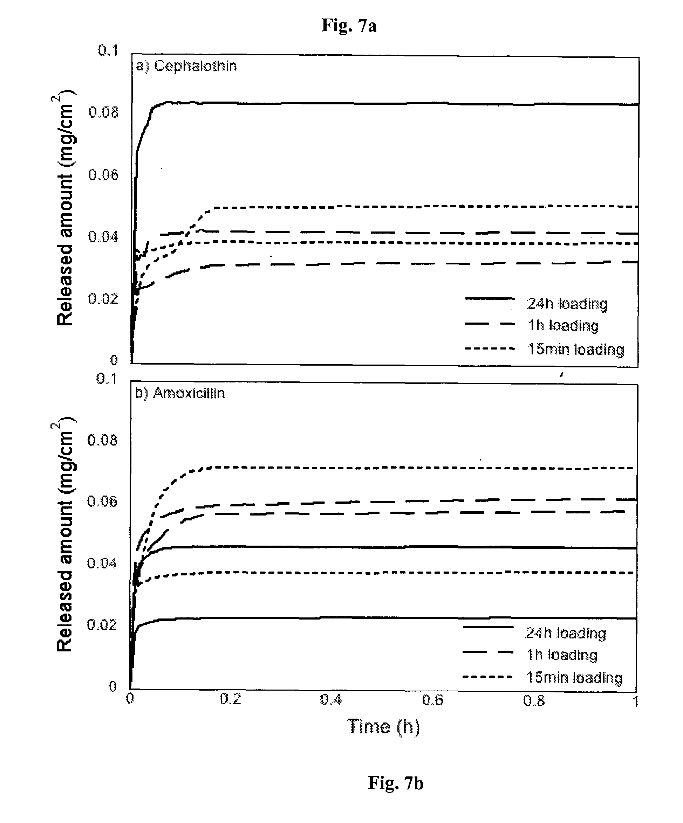

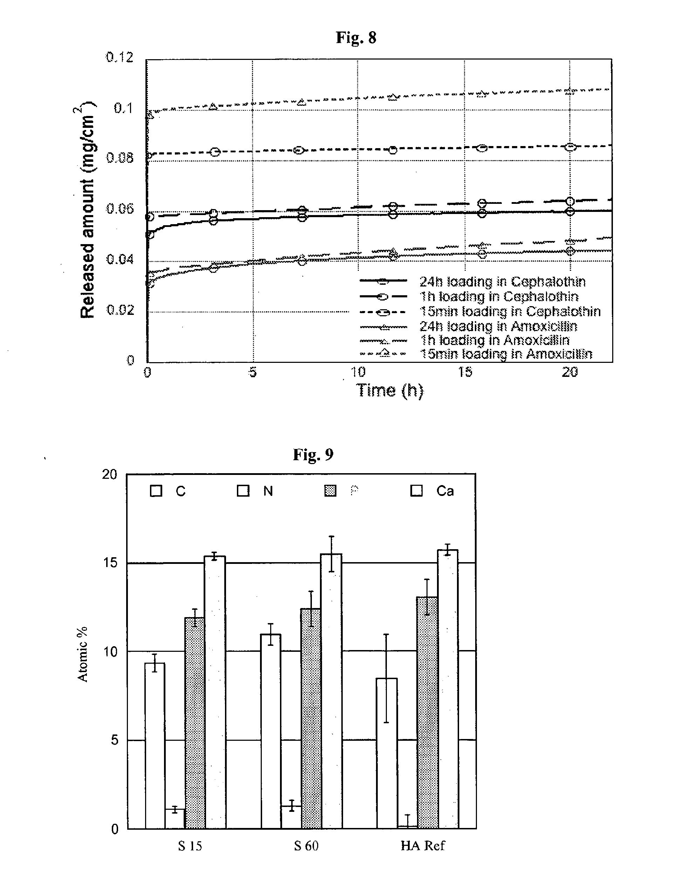

Figs. 7a and 7b show examples of the initial drug release in water from HA coatings loaded with Cephalothin and Amoxicillin, respectively, at three different loading times. As is apparent, an initial rapid drug release, hereafter referred to as the burst effect, varied in amount and did not show any clear dependence on drug loading time. In all experiments, the initial burst process lasted for less than 10 minutes, after which a slow and reproducible release was detected. This slow release was found to be independent of drug loading time and somewhat faster for Amoxicillin than for Cephalothin.
In order to study the slow release process in more detail, similar experiments to those described in connection with Fig. 6 were carried out for 22 hours in PBS, results of which are shown in Fig. 8. For these measurements, the amount of drug released during the initial burst period was found to be erratic and not related to the drug loading time. The slow release process following the burst was, however, again found to be reproducible and rather similar for all drug loading times studied, with the 15 minutes and 1 hour loading processes resulting in the most linear release processes. These findings suggest that the implants preferably should be stored for about 10 minutes in PBS after drug loading to ensure reproducibility in the subsequent release process.
The drug release rate of Cephalothin was found to be $0.22\pm0.06$ $\mu$g/h·cm$^2$ while the corresponding release rate for Amoxicillin was found to be $0.42\pm0.10$ $\mu$g/h·cm$^2$ between 2 and 22 hours after the experiment was started. The slower release rate for Cephalothin as compared to Amoxicillin is in accordance with earlier findings showing that the former molecule binds more strongly to HA.
Table 1 presents the concentration of Amoxicillin and Cephalothin that has to be present to ensure killing of *S. aureus* and *S. epidermidis*, as divulged from the antimicrobial susceptibility tests.

Table 1: Result from E-test for antimicrobial susceptibility testing, determining the minimum inhibitory concentration (MIC) of antimicrobial agents against microorganisms (inhibiting bacteria in 1 ml broth).

| Antibiotic drug | Bacteria | |
| --- | --- | --- |
| | *Staphylococcus aureus,* MIC ($\mu$g/ml) | *Staphylococcus epidermidis,* MIC ($\mu$g/ml) |
| Amoxicillin | 0.064 | 0.19 |
| Cephalothin | 0.064 | 0.064 |

Comparing the release rates of the slow release from the implant coatings with the doses necessary to accomplish bacteria killing according to the antimicrobial susceptibility tests demonstrates that during the slow release process from the implant coating, a sufficient dose to ensure killing of *S. aureus* in a radius of 1 cm from the implant is released every 17 minutes from the Cephalothin loaded coatings whereas the corresponding time is about 9 minutes from the Amoxicillin loaded ones. A sufficient dose to ensure killing of *S. epidermidis* in the corresponding region is released every 17 minutes from the Cephalothin loaded coatings and every 27 minutes from the Amoxicillin loaded ones. These numbers are calculated assuming that the transport of drugs from the region in vicinity of the implant is slow. If the drug release rate from the implant surface is sufficient to maintain an antibacterial effect close to the implant in vivo depends on the speed of the process transporting the antibiotics away from the region close to the implant as well as on how the drug release process is affected by other release conditions than those prevailing in our experiments.

After the release experiments in PBS, two of the HA coatings that had been loaded for 24 hours were dissolved in hydrochloric acid to determine the amount of antibiotics remaining after 22 hours of release. We found that about 3.5 times more Cephalothin (~18 $\mu$g) was present in the coatings as compared to Amoxicillin (-5.0 $\mu$g). These amounts can be used to obtain a prediction of the expected total release time from the investigated coatings. Assuming that the constant release rate of 0.22 (Cephalothin) and 0.42 (Amoxicillin) $\mu$g/h·cm$^2$ found during the first day of release is upheld until the coatings are emptied, the amounts found to be remaining in the 2 cm$^2$ hydrochloric acid treated coatings give an anticipated release time of between 27 and 63 hours (including the 22 hours of release before the hydrochloric acid treatment) for Amoxicillin and Cephalothin, respectively. These numbers are of course estimates, but they still give an indication on the state of the art from which the HA coating stoichiometry, porosity and thickness as well as the drug loading process should be optimized when longer release times are aimed for.

This example demonstrate a method to fast-load biomimetic hydroxyapatite surgical implant coatings with antibiotics in order to create the platform required for a multifunctional implant device which offers sustained local antibacterial treatment, avoiding systemic drug administration, and which also avoids problems with safe drug handling and storage, and gives the surgeon the possibility to choose which antibiotics or other active agent to incorporate in the implant at the site of surgery. The titanium substrate surfaces were coated with an adhesion enhancing layer of Ti-oxide, having a gradient in the oxygen content and resulting in bioactive crystalline anatase TiO$_2$ on the outermost surface, using physical vapor deposition. On this bioactive layer hydroxyapatite was biomimetically grown to serve as a combined bone in-growth promoter and drug delivery vehicle. Four antibiotic drugs, Amoxicillin, Gentamicin sulfate, Tobramycin and Cephalothin, were loaded into the implant coatings via soaking at different time periods between 15 minutes and 24 hours. The subsequent release from Cephalothin and Amoxicillin loaded samples was studied using UV spectroscopy whereas the antibacterial effect of samples containing all 4 types of antibiotics was assessed by examination of inhibition zones around samples placed in a *Staphylococcus aureus* containing agar after having been allowed to release their antibiotic content for various time periods in a PBS solution.

The antibacterial tests showed that even after short drug loading times of 15 minutes, all four types of antibiotic loaded samples released enough drugs after 24 hours to ensure bacterial killing in the vicinity of the samples. From the UV measurements it was found that the release processes of Cephalothin and Amoxicillin consisted of an initial rapid (less than 10 minutes) drug release which varied in amount followed by a sustained release process with a reproducible release rate found to be rather independent of the loading times used. The latter process was somewhat faster for Amoxicillin than for Cephalothin. These findings demonstrate that implants that have been fast-loaded with drugs according to the process disclosed herein should be maintained for about 10 minutes or another time period determined by one skilled in the art in a simulated body fluid like PBS after loading to ensure reproducibility in the subsequent release process.

The calculated release rates and measurements of drug amounts remaining in the samples after 22 hours of release indicated that a therapeutically relevant dose could be achieved close to the implant surface for about 2 days. In cases when a more prolonged release is aimed for, the porosity, stoichiometry and thickness of the HA can be optimized in several ways by one skilled in the art, e.g., by changing the solution temperature and deposition time of the biomimetic process as well as the Ca content of the PBS buffer used. As well, the drug loading process can be tuned by, e.g., changing the concentration of the drugs in the soaking solution, changing the temperature or the dissolution medium (e.g. increase or reduction in pH, using ethanol and/or acetone).

This example therefore provides a method for a fast-loading slow-release surgical implant kit where the implant and the drug are separated when delivered to the surgeon, thus avoiding various problems related to a lack of knowledge of drug handling at implant production and relating to sterilization of implants containing drugs prior to delivery. Most importantly, however, the present methods offer the choice of quick addition of antibiotics or other active agents to an implant coating, thus constituting a flexible solution for the surgeon.

Example 9

This example demonstrates the incorporation of a bisphosphonate in implant coatings. Bisphosphonates (BPs) constitute a class of drugs which has been used since the middle of the 20th century for treatment of bone diseases caused by increased bone resorption such as osteoporosis, Paget's disease and tumor bone disease. BPs contribute to making the bone structure stronger by increasing the rate of cell death among the osteoclasts. Studies have also indicated that BPs also have a positive effect on the osteoblasts. These properties of BPs have made them interesting for use in connection with bone implants. A more effective bone generation gives a stronger bone structure around the implant which decreases the risk of implant loosening. In animal studies, a positive effect from BP on the bone generation around implants has been observed.

BPs can be administered orally or subcutaneously. Both demand higher dose concentrations than locally delivered BPs, which may be achieved by loading of BPs into the surface of an implant before implantation. Various loading methods for implant surfaces have been suggested, many of which rely on the presence of calcium in the surface layer since BPs have a high affinity to calcium. Calcium ions can either be implanted onto a titanium surface, thus immobilizing the BPs on the surface, or BPs can be loaded into a Ca-containing hydroxyapatite (HA) coating. Granules of HA have been studied for the loading into and the subsequent release of BPs. In these studies, the loading was performed by soaking the samples in a solution of BPs dissolved in water. When a bioactive surface is soaked in simulated body fluid (SBF) or phosphate buffer saline (PBS), a HA coating is formed on the surface from the constituents of SBF. Attempts to load the HA layer with BPs have been made, either via soaking into the HA layer or via co-precipitation onto the surface. See, for example, Mcleod et al, "Adsorption of bisphosphonate onto hydroxyapatite using a novel co-precipitation technique for bone growth enhancement," J. Biomedical Materials Research, 79A(2);271-281 (Nov 2006), which compared water with SBF as a dissolution media for BPs. In this study, they discovered a higher BP content in the sample soaked in SBF than in the sample soaked in water, when analyzed with Electron Spectroscopy for Chemical Analysis (ESCA). From this they drew the conclusion that BPs were precipitated simultaneously with HA in the sample soaked in SBF, not only adsorbed onto the surface. Cell culture studies revealed that the effect on osteoclasts was the same for both samples. To form a biomimetic HA layer into which pharmaceutically active ingredients can be loaded may be difficult, especially on metals and polymers. Normally this is done via chemical treatment of the surface or extensive soaking times.

As an alternative method, according to the present invention, the implant is provided with a more bioactive surface chemistry, i.e. a bioactive surface of titanium oxide of increased crystallinity.

Since the HA on implants is resorbed after implantation, inclusion of BPs in the HA will allow a continuous local administration of BPs until full bone in growth has been completed, giving a strong bone structure around the implant. If an implant as manufactured contains BPs, a surgeon will not have the option to include or exclude the drug based on the patient need. In some cases, the risk of infection is large that antibiotics are needed, instead of or in combination with BPs. Therefore, to increase the range of options for the clinicians, the present methods allowing the implant to be loaded with drugs in a short time with a procedure that can be carried out during or immediately before surgery is very valuable.

In the present example we demonstrate a method for fast loading by soaking of BPs in HA-coatings formed on a composite material according to the invention. Fast loading allows the clinical user to load the implant directly in the operating room.

*Sample preparation*

Gradient titanium dioxide thin films were prepared in a reactive DC magnetron sputtering unit (Balzer UTT400) at 350°C. The coatings were deposited on 20 x 20 mm plates of commercially pure grade 5 titanium by PVD. The working chamber was mounted with a turbo molecular pump TMU 521P. The chamber base pressure was $\sim 10^{-7}$ mbar after backing. Pure titanium (99.9%) was used as target, and pure argon (99.997%) and oxygen (99.997%) were used for the reactive sputtering. The substrate was kept at a constant temperature of 350°C during the entire process.

The oxygen profile of the film can be controlled by changing the oxygen flow. An increasing oxygen flow reduces the metallic content. A crystalline titanium dioxide was ensured by heat treatment of the sputtered film at 385°C in air for one hour post deposition. The targeted coating design parameters were as follows, described from the titanium substrate of grade 5: 50 nm of pure titanium, 50 nm gradually increasing oxygen content from Ti to $TiO_2$ and a top layer of 100 nm crystalline $TiO_2$. The thickness of the films was measured with surface profilometry, using a Tencor Alpha-step 200 instrument (USA product). The test plates were ultrasonically cleaned, at resonance of 20 kHz, by the following procedure: 5 minutes in 100 ml of acetone, 5 minutes in ethanol, and finally 5 minutes in deionized water.

*Loading of pamidronate*

The bisphosphonate was pamidronate disodium salt hydrate, $C_3H_9NO_7P_2Na_2 \cdot xH_2O$ (Sigma Aldrich, 95%). The titanium plates were soaked in 10 ml of PBS (Dulbecco, containing $CaCl_2$, $MgCl_2$, KCl, $KH_2PO_4$, NaCl and $Na_2HPO_4$, Sigma-Aldrich) for seven days at 40°C to allow for a homogenous HA layer to form on the surface. Next, the samples were removed from the solution, gently rinsed in distilled water and air dried. Thereafter, the samples were soaked in 10 ml of a solution of pamidronate in deionized water, (0.5 mg/ml), for 15 minutes (samples S15) or 1 hour (samples S60). After soaking, the samples were air dried, then stored in Petri dishes at room temperature.

Four plates (samples HA-Ref) were soaked in PBS for seven days at 40 °C without being exposed to pamidronate to be used as reference samples. Additionally, a pamidronate reference sample (denoted Pam) was prepared by depositing an aqueous solution of pamidronate (10 mg/ml) on one of the titanium plates. Thereafter, the sample was placed in 40 °C until the water had evaporated.

*ESCA analysis*

A full elemental analysis of the surface was done using a Physical Electronics Quantum 2000 spectrometer with a monochromatic A1 K $\alpha$ radiation. The diameter of the area of analysis was set to 100 $\mu$m. Pass energy was set to 187.85 eV and a step size of 0.8 eV was used. Spectra were acquired for the following peaks; C1s, Ca2p, N1s, O1s P2p and Ti2p after each sputter cycle of 0.2 minutes. Nitrogen and carbon were used as indicators for presence of pamidronate since these are the only two elements that are present in pamidronate but not in HA. The positions in energy of the ESCA peaks were shifted with respect to that of oxygen 1s at 531 eV to minimize errors due to charging of the surface during analysis.

Fig. 9 shows the atomic concentrations measured on the samples prepared using the fast loading method, together with the results from the reference HA sample. As expected, nitrogen was present on the samples soaked in the pamidronate-containing solution. The reference sample had amounts of nitrogen that are below the limit of detection of the instrument. 15 minutes of soaking yielded a nitrogen content of 1.1 $\pm$ 0.1 % which was only slightly less than the sample soaked for 60 minutes, 1.3 $\pm$ 0.1. The atomic percentage of carbon and phosphor followed the same trend as nitrogen. On samples expected to contain pamidronate, there was more phosphor than calcium as opposed to the HA-Ref.

*Scanning Electron Microscopy (SEM)*

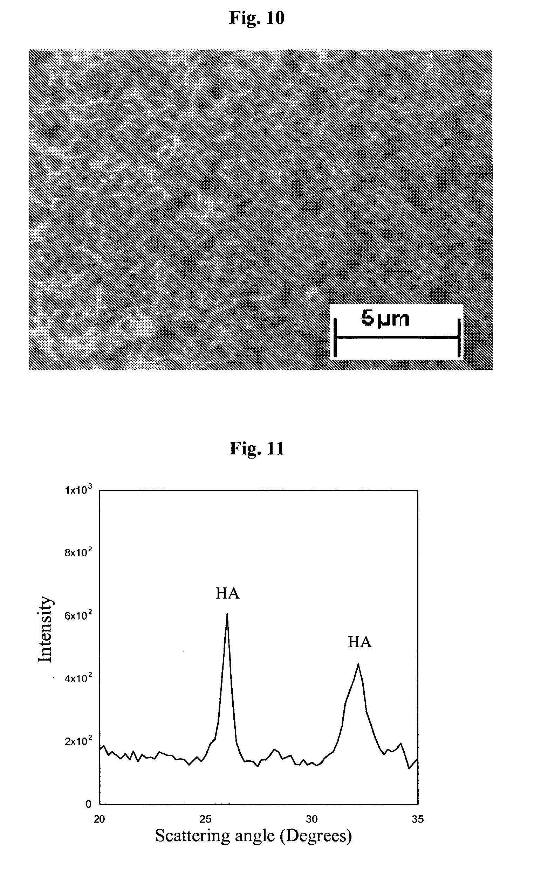

The surface structure of the samples was examined with a LEO 1550 equipped with a field emission gun. An in-lens detector was used and the acceleration voltage was set to 3.35 kV. During SEM analysis, energy dispersive x-ray analysis (EDX) was performed for element analysis. The surfaces of the samples were homogenously covered with a layer of HA crystals which was formed during the soaking in PBS, as shown in Fig. 10 showing a SEM image of the S60 surface. Similar structures were observed on the S15 and HA-Ref samples as well.

*X-Ray diffraction (XRD)*

To verify the formation of HA-crystal on the samples after the preparation, XRD measurements of the coatings were performed using a Siemens D5000 diffractometer. The grazing incidence angle was 0.2 degree in parallel beam geometry using CuK$\alpha$ radiation (wavelength 1.540598 Å). Collection of diffracted x-rays was done between 20 and 42 degrees for 2 seconds every 0.2 degree. XRD analysis of the samples showed clear presence of HA as seen in Fig. 11. The peaks at 26° and 32° are characteristic for HA.

*Alternating ionic current (AIC) conductivity measurements*

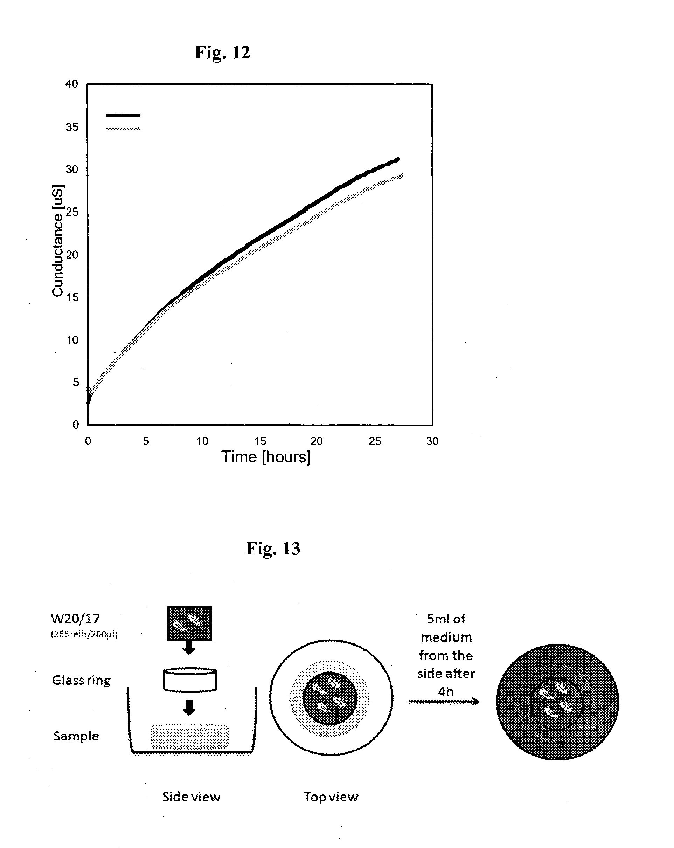

The AIC technique is a method developed to measure release of ionic drugs from a host matrix. Since pamidronate is ionic, AIC was used to detect its release. The samples were placed in the AIC-cell and immersed in 20 ml of distilled water. A small magnet stirrer was used at low speed to ensure a continuous motion in the liquid. Measurements were performed during 24 hours. Fig. 12 shows the conductance curves of S60 and HA-Ref. Overall, the measured conductance in the solution surrounding both samples was extremely low, indicating that the total amount of ions released from the coatings was very low. The curves closely follow each other indicating that there was no significant difference in the amount of released ions from the surface of the two samples. After ~ 10 hours, the curves slightly separate, but as seen in the figure, the maximum difference between them is less than the conductance level at time zero signifying the conductivity of deionized water. This demonstrates that the pamidronate present in the fast-loaded samples is strongly bound to the HA structure.

This example demonstrates fast loading by soaking in a pamidronate solution for 15 minutes proved to be sufficient to load pamidronate into a hydroxyapatite coating. The pamidronate that is loaded into the hydroxyapatite coating becomes tightly bonded to the hydroxyapatite structure. The release of pamidronate and other bisphosphonates after implantation is thus expected to occur with the same pace as HA is resorbed, i.e. over several weeks.

Example 10

In the present example, the coating described in Example 8 is loaded with two functional substances, one that is released (Amoxicillin) and one that is strongly bound in the coating (bisphosphonate) and only made available for uptake by the body when the HA layer is resorbed. The incorporation of active substances is facilitated by increasing the temperature of the solution containing the pharmaceutical ingredients during loading of the implant. After an implant with the described coating is placed inside the body by a surgeon, the antibiotics is thus released for several days while the bisphosphonate functionalized surface prevents osteoclast formation on the surface for a prolonged period of time. The fast loading process was carried out in the following manner: a substrate including an HA coating as described in Example 8 was soaked in a solution containing 10 ml PBS, 6 mg of pamidronate disodium salt hydrate, and 100 $\mu$g Amoxicillin at 50 °C for 25 minutes. The sample was thereafter rinsed for 5 minutes in PBS and characterized using the same methods as in Examples 8 and 9. The characterization showed that a therapeutically significant dose of amoxicillin was released for 7 days from the implant coatings whereas the bisphosphonate was sufficient in concentration to promote bone in growth.

Example 11

In the present example, the same pharmaceutical ingredients as in Example 10 were loaded into the HA coating of the composite material described in Example 8, except that in this example, the bisphosphonate was loaded first, followed by the amoxicillin in the following manner: The HA coating of Example 8 was soaked in a solution containing 10 ml PBS, and 7 mg of pamidronate disodium salt hydrate at 40°C for 12 minutes. Thereafter the sample was soaked in a solution containing 10 ml PBS, and 70 $\mu$g Amoxicillin at 40°C for 15 minutes. The sample was thereafter soaked in PBS for 7 minutes and characterized using the same methods as in Examples 8 and 9. The characterization showed that a therapeutically significant dose of amoxicillin was released for 6 days from the implant coatings whereas the bisphosphonate was sufficient in concentration to promote bone in growth.

Example 12

In the present example, composite materials as described generally in Example 8 were prepared except that the structure of the HA coating in Example 8 was changed in several ways. First, a thicker HA coating was formed by increasing the time the $TiO_2$ coating in Example 8 was stored in PBS from 4 to 6 days at 60°C (Sample 1). Secondly, a more porous HA coating was obtained by storing the $TiO_2$ coating in Example 8 for 4 days at room temperature (Sample 2). Thirdly, the Ca content of the PBS buffer from Example 8 was increased by 20 % and the $TiO_2$ coating in Example 8 was stored for 4 days at 60°C (Sample 3). The drug loading procedure in Example 10 with subsequent characterization was repeated for these 3 HA coatings, with the following results.

Sample 1: After soaking in the same solution as in Example 10 containing PBS, Pamidronate disodium salt hydrate and Amoxicillin, Sample 1 was shown to contain 50% more Amoxicillin than the coating in Example 10 whereas the amount of bisphosphonate on the surface was similar to the amount in Example 10. The release of Amoxicillin studied by UV spectroscopy under the same conditions as in Example 8 was found to take place for 1.5 days longer than that in Example 10.

Sample 2: After soaking in the same solution as in Example 10 containing PBS, Pamidronate disodium salt hydrate and Amoxicillin, Sample 2 was shown to contain 20% more Amoxicillin than the coating in Example 10 even though the thickness of the coating was found to be 30% smaller. The larger porosity of the HA created at a lower biomimetic precipitation temperature is believed to be the cause of this. The amount of bisphosphonate on the surface was similar to the amount in Example 10.

Sample 3: After soaking in the same solution as in Example 10 containing PBS, Pamidronate disodium salt hydrate and Amoxicillin, Sample 3 was shown to contain more Ca than the otherwise Ca deficient HA that normally forms during HA growth.

Example 13

This example demonstrates a cell proliferation evaluation. Three different samples were used in a cell proliferation experiment. Sample A was commercially pure grade 2 titanium with native amorphous $TiO_2$ on the surface. Sample B was sputter coated using the procedure outlined in Example 8 to create crystalline $TiO_2$ surfaces. Grade 2 titanium plates were used as substrates for Sample B. Sample C was made from some of the Sample Bs that were submerged in a phosphate buffer at 40°C for 1 week to grow a layer of hydroxyapatite on the surface. All samples were sterilized at by autoclaving (120°C for 30 min).

Samples were placed at the bottom of a six well plate and covered with a sterile glass ring as schematically shown in Fig. 13. W20-17 cells (mesenchymal stem cells from ATCC) were seeded (100,000 cells/100 $\mu$l medium) on top of each sample. The cells were allowed to attach at 37°C for 4h, after which 5 ml of medium was added from the side to each well. The glass rings were then removed and the cells were cultured for 2 and 7 days and the cell proliferation was evaluated by an MTT assay.

A standard curve was prepared from the same cell suspension (0, 5E4, 1E5, 2E5, 6E5, 1E6 in 5 ml DME HAM:F12 medium). MTT was performed on the standard curve after 0 h. 500 $\mu$l of 5 mg/ml sterile MTT solution was added in each well and the plates were incubated for 4h at 37°C, 5% $CO_2$. Then the medium was removed and the crystals were dissolved in 5 ml DMSO. 200 $\mu$l from each sample was transferred to a 96-well plate and detected at 570 nm. The number of cells was determined from a standard curve.

The average number (over 3 measurements) of cells viability after 2 and 7 days respectively are given in the below Tables 2 and 3.

Table 2: Number of living cells after 2 days on the different substrates

| Samples | Sample A | Sample B | Sample C |
|---|---|---|---|
| Average | 83958 | 93722 | 110563 |

Table 3: Number of living cells after 7 days on the different substrates

| Samples | Sample A | Sample B | Sample C |
|---|---|---|---|
| average | 49306 | 91681 | 127708 |

The above results show the clear trend that the cell proliferation is higher on crystalline $TiO_2$ and HA than on the native $TiO_2$ formed on the commercially pure titanium substrate.

Example 14

This example demonstrates a cell differentiation evaluation. Similar Samples A and B as described in Example 13 were used. In addition, a Sample D was made by soaking Sample C as described in Example 13 in a solution containing sterile water and 1 $\mu$g/ml of the bone morphogenetic protein BMP-2 (Wyeth Lederle) for 30 minutes at 4 °C.

W20-17 cells were seeded on samples in 24 well plates with a glass ring on the sample (100,000 cells/200 $\mu$l). After 4h 2 ml of medium was added. The rings were left to stay and the samples were incubated at 37°C, 5% $CO_2$ for 24 and 48 hours.

ALP assay 24h: Cell culture medium was aspirated from the wells and samples were washed once with PBS. Thereafter, 500 $\mu$l distilled water was added and the cells were lysed by repeated freezing and thawing (30 min at -80°C, 15 min at 37°C, twice) to release the ALP into solution. In the meantime, the ALP solution was prepared in 10 ml of distilled water: 50 mM glycine, 0,05 % Triton X-100, 4 mM $MgCl_2$ and 5 mM p-nitrophenyl phosphate.

The pH was adjusted to 10.3 and 1 ml was added to each well. The plates were once again incubated at 37°C for 20 min, before adding 500 $\mu$l 0.1 NaOH to stop the reaction. 200 $\mu$l portions from each sample were transferred to a 96-well plate and detected at 405 nm.

ALP assay 48h: The ALP expression was detected as for the 24h time point.

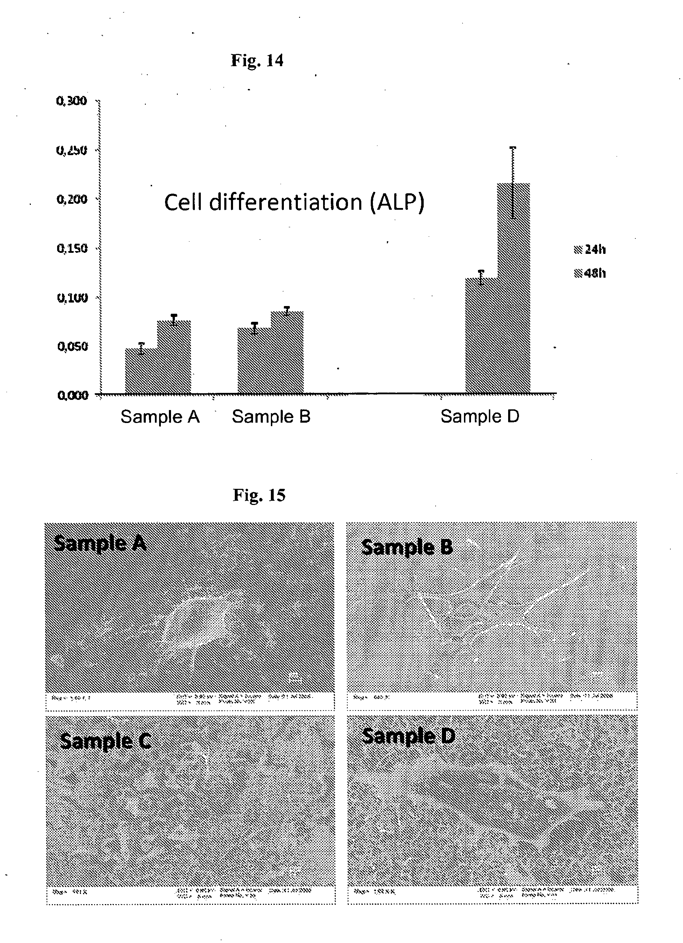

The results show successful adsorption of BMP-2 to the HA surface. From both the ALP 24h and the ALP 48h experiments, shown in Fig. 14, it appears that crystalline $TiO_2$ (Sample B) induces a higher level of differentiation than the native amorphous $TiO_2$ (Sample A). The cell differentiation is dramatically improved by loading BMP-2 into the HA containing layer of Sample D.

Example 15

This example evaluates cell morphology. Samples A, B, C and D described in Examples 13 and 14 were used.

W20-17 cells were seeded on samples in a 24 well plate with a glass ring on the sample (100,000 cells/200 $\mu$l). After 4h, 2 ml of medium was added. The rings were left to stay and the samples were incubated at 37°C, 5% $CO_2$ for 2 days.

Fixation and Dehydration: The samples were washed once with PBS, placed in 2.5% glutaraldehyde/PBS solution, left for 2 hours, placed in 50% EtOH for 10 min, and left in 75% EtOH overnight. The next day, the samples were rinsed in 90% EtOH for 1.5h, then in 100% EtOH for 1h. The samples were allowed to air-dry and were kept in a desiccator until they were analyzed with SEM.

SEM imaging: The samples were sputtered with Au/Pa and imaged. As is obvious from the SEM images in Fig. 15, the cells are significantly more spread out over the surface on the crystalline $TiO_2$ (Sample B) and the two HA covered samples (Samples C and D) as compared to the surface with native $TiO_2$ (Sample A), indicating that they are thriving in Samples B-D.

The specific illustrations and embodiments described herein are exemplary only in nature and are not intended to be limiting of the invention defined by the claims. Further embodiments and examples will be apparent to one of ordinary skill in the art in view of this specification and are within the scope of the claimed invention.

**Claims**

1. A method of forming a surgical implant composite material, comprising depositing a thin film coating on a surgical implant substrate, the thin film coating comprising $TiO_{2-x}M_y$, wherein M is one or more elements which does not adversely effect adherence of the coating to the substrate, y is the sum of the mols of all M elements, $0 \leq x < 2$ and $0 \leq y \leq 1$, and wherein an outermost portion of the thin film coating is crystalline, and heating the deposited thin film coating at a temperature sufficiently high to increase the crystalline grain size in the thin film coating and further comprising a step of forming a biomimetic coating comprising calcium phosphate on the thin film coating by a biomimetic precipitation from a buffer solution comprising calcium phosphate.

2. A method according to claim 1, wherein the crystalline grains in the thin film coating are larger than 1 nm, 5 nm, 10 nm, 20 nm, 30 nm, 35 nm, 40 nm, 45 nm or 50 nm.

3. The method of claim 2, wherein the heating step is conducted at a temperature less than 400°C.

4. A method according to claim 1, wherein the substrate is metallic and the method further comprises cleaning and sputter etching the substrate surface to remove native oxide prior to depositing the thin film coating thereon.

5. A method according to claim 1, wherein the thin film coating is deposited by physical vapor deposition technique or chemical vapor deposition technique.

6. A method according to claim 1, further comprising loading the biomimetic coating with a releasable agent comprising an active pharmaceutical ingredient, ion or bio molecule, or a combination thereof.

7. A method according to claim 1, wherein the thin film coating has a gradient composition through at least a portion of the thin film coating thickness.

8. A method of preparing a surgical implant composite material for implantation in an individual, comprising selecting a releasable agent to be loaded on the surgical implant composite material based on a condition of the individual, and contacting the surgical implant composite material with a solution of the releasable agent to load the releasable agent onto the surgical implant composite material, the surgical implant composite material comprising a surgical implant substrate and a thin film coating deposited on the substrate, the thin film coating comprising $TiO_{2-x}M_y$, wherein M is one or more elements which does not adversely effect adherence of the coating to the substrate, y is the sum of the mols of all M elements, $0 \leq x < 2$ and $0 \leq y \leq 1$, and wherein an outermost portion of the thin film coating is crystalline, with crystalline grains larger than 1 nm, wherein the surgical implant composite material further comprises a step of forming a biomimetic coating comprising calcium phosphate on the thin film coating by a biomimetic precipitation from a buffer solution comprising calcium phosphate and wherein the releasable agent is loaded onto the biomimetic coating and the releasable agent comprising an active pharmaceutical ingredient, ion or bio molecule, or a combination thereof.

9. A method according to claim 1 or claim 8, wherein M is selected from the group including Ag, I, Si, Ca, Zr, Hf, P, C and N or any combination thereof.

**Patentansprüche**

1. Verfahren zum Bilden eines Verbundmaterials für ein chirurgisches Implantat, welches ein Abscheiden einer Dünnschichtbeschichtung an einem Substrat für ein chirurgisches Implantat umfasst, wobei die Dünnschichtbeschichtung $TiO_{2-x}M_y$ aufweist, wobei M ein oder mehrere Elemente sind, welche die Haftfestigkeit der Beschichtung an dem Substrat nicht nachteilig beeinflussen, y die Summe der Mole aller M-Elemente ist, $0 \leq x < 2$ und $0 \leq y \leq 1$, und wobei ein äußerster Teilbereich der Dünnschichtbeschichtung kristallin ist, sowie ein Erwärmen der abgeschiedenen Dünnschichtbeschichtung bei einer Temperatur, die ausreichend hoch ist, so dass die kristalline Korngröße in der Dünnschichtbeschichtung zunimmt, und welches ferner einen Schritt des Bildens einer Calciumphosphat aufweisenden biomimetischen Beschichtung an der Dünnschichtbeschichtung mittels einer biomimetischen Abscheidung aus einer Calciumphosphat aufweisenden Pufferlösung umfasst.

2. Verfahren nach Anspruch 1, wobei die kristallinen Körner in der Dünnschichtbeschichtung größer als 1 nm, 5 nm, 10 nm, 20 nm, 30 nm, 35 nm, 40 nm, 45 nm oder 50 nm sind.

3. Verfahren nach Anspruch 2, wobei der Schritt des Erwärmens bei einer Temperatur von weniger als 400°C ausgeführt wird.

4. Verfahren nach Anspruch 1, wobei das Substrat metallisch ist und das Verfahren ferner ein Reinigen und Sputterätzen der Substratoberfläche umfasst, so dass natürliches Oxid vor dem Abscheiden der Dünnschichtbeschichtung auf der Substratoberfläche entfernt wird.

5. Verfahren nach Anspruch 1, wobei die Dünnschichtbeschichtung mittels physikalischer Gasphasenabscheidung oder chemischer Gasphasenabscheidung abgeschieden wird.

6. Verfahren nach Anspruch 1, welches ferner ein Beaufschlagen der biomimetischen Beschichtung mit einem lösbaren Mittel umfasst, welches einen aktiven pharmazeutischen Bestandteil, ein Ion oder Biomolekül oder eine Kombination derselben aufweist.

7. Verfahren nach Anspruch 1, wobei die Dünnschichtbeschichtung eine sich ändernde Zusammensetzung über zumindest einen Teilbereich der Dicke der Dünnschichtbeschichtung aufweist.

8. Verfahren zum Bereitstellen eines Verbundmaterials für ein chirurgisches Implantat zum Implantieren in ein Individuum, welches ein Auswählen eines lösbaren Mittels umfasst, das vorgesehen ist zum Beaufschlagen des Verbundstoffs für ein chirurgisches Implantat, basierend auf einer Bedingung des Individuums sowie ein Inkontaktbringen des Verbundstoffs für ein chirurgisches Implantat mit einer Lösung des lösbaren Mittels, so dass der Verbundstoff für ein chirurgisches Implantat mit dem lösbaren Mittel beaufschlagt wird, wobei der Verbundstoff für ein chirurgisches Implantat ein Substrat für ein chirurgisches Implantat und eine Dünnschichtbeschichtung umfasst, die auf dem Substrat abgeschieden ist, wobei die Dünnschichtbeschichtung $TiO_{2-x}M_y$ aufweist, wobei M ein oder mehrere Elemente sind, welche die Haftfestigkeit der Beschichtung an dem Substrat nicht nachteilig beeinflussen, y die Summe der Mole aller M-Elemente ist, $0 \leq x < 2$ und $0 \leq y \leq 1$, und wobei ein äußerster Teilbereich der Dünnschichtbeschichtung kristallin ist, mit kristallinen Körnern größer als 1 nm, wobei der Verbundstoff für ein chirurgisches Implantat ferner einen Schritt des Bildens einer Calciumphosphat aufweisenden biomimetischen Beschichtung an der Dünnschichtbeschichtung mittels biomimetischer Abscheidung aus einer Calciumphosphat aufweisenden Pufferlösung umfasst, und wobei die biomimetische Beschichtung mit dem lösbaren Mittel beaufschlagt wird und das lösbare Mittel einen aktiven pharmazeutischen Bestandteil, ein Ion oder Biomolekül oder eine Kombination derselben aufweist.

9. Verfahren nach Anspruch 1 oder Anspruch 8, wobei M aus der Gruppe, welche Ag, I, Si, Ca, Zr, Hf, P, C und N oder eine beliebige Kombination derselben umfasst, ausgewählt wird.

**Revendications**

1. Un procédé pour former un matériau composite pour implant chirurgical, comprenant

- le dépôt d'un revêtement formé d'un film mince sur un substrat pour implant chirurgical, dans lequel le revêtement est formé d'un film mince comprenant du $TiO_{2-X}M_Y$,
formule où

~ M est un ou plusieurs éléments qui n'affectent pas défavorablement l'adhérence du revêtement au substrat,
~ y est égal à la somme des moles de tous les éléments M,
~ $0 \leq x < 2$ et
~ $0 \leq y \leq 1$,

dans lequel une portion la plus externe du revêtement formé d'un film mince est sous forme cristalline,
- et le chauffage du revêtement sous forme de film mince déposé à une température suffisamment élevée pour augmenter la dimension du grain cristallin dans le revêtement formé d'un film mince et comprenant en outre l'étape consistant à former un revêtement biomimétique comprenant du phosphate de calcium sur le revêtement de film mince par précipitation biomimétique à partir d'une solution tampon comprenant du phosphate de calcium

2. Un procédé selon la revendication 1, dans lequel les grains cristallins dans le revêtement formé d'un film mince présentent une dimension supérieure à 1 nm, 5 nm, 10 nm, 20 nm, 30 nm, 35 nm, 40 nm, 45 nm ou 50 nm.

3. Le procédé selon la revendication 2, dans lequel l'étape de chauffage est conduite à une température inférieure à 400°C.

4. Le procédé selon la revendication 1, dans lequel le substrat est métallique et le procédé comprend en outre le nettoyage et la gravure par pulvérisation cathodique de la surface du substrat pour éliminer l'oxyde natif avant le dépôt sur celle-ci du revêtement formé d'une couche mince.

5. Procédé selon la revendication 1, dans lequel le revêtement formé d'une couche mince est déposé par une technique de dépôt physique en phase vapeur ou une technique de dépôt chimique en phase vapeur.

6. Procédé selon la revendication 1, comprenant en outre le chargement du revêtement biomimétique avec un agent libérable comprenant un ion, une biomolécule, un principe actif pharmaceutique, ou une combinaison de ceux-ci.

7. Procédé selon la revendication 1, dans lequel le revêtement formé d'une couche mince présente un gradient de composition pour au moins une partie de l'épaisseur du revêtement de film mince.

8. Procédé de préparation d'un matériau composite pour implant chirurgical destiné à être implanté chez un individu, comprenant la sélection d'un agent libérable à charger sur le matériau composite pour implant chirurgical en fonction de l'état de l'individu, et le contact du matériau composite pour implant chirurgical avec une solution de l'agent libérable pour charger l'agent libérable sur le matériau composite pour implant chirurgical, le matériau composite pour implant chirurgical comprenant un substrat pour implant chirurgical et un revêtement formé d'un film mince déposé sur le substrat, le revêtement formé d'un film mince comprenant $TiO_{2-X}$-$M_Y$, M est un ou plusieurs éléments qui n'affectent pas défavorablement l'adhérence du revêtement sur le substrat, y est égal à la somme des moles de tous les éléments de M, $0 \leq x < 2$ et $0 \leq y \leq 1$, et dans lequel une portion la plus externe du revêtement formé d'un film mince est sous forme cristalline, formée de grains cristallins supérieurs à 1 nm, dans lequel le matériau composite pour implant chirurgical comprend en outre l'étape consistant à former un revêtement biomimétique comprenant du phosphate de calcium sur le revêtement de film mince par précipitation biomimétique à partir d'une solution tampon comprenant du phosphate de calcium et dans lequel l'agent libérable est chargé dans le revêtement biomimétique et l'agent libérable comprend un ion, une biomolécule, un principe actif pharmaceutique, ou une combinaison de ceux-ci.

9. Procédé selon la revendication 1 ou la revendication 8, dans lequel M est choisi dans le groupe comprenant Ag, I, Si, Ca, Zr, Hf, P, C et N ainsi que de toutes combinaisons de ceux-ci.

**Fig. 1**

**Fig. 2**

**Fig. 3a**

**Fig. 3b**

**Fig. 4**

**Fig. 5**

**Fig. 6**

**Fig. 7a**

**Fig. 7b**

## Fig. 8

## Fig. 9

Fig. 10

5 µm

Fig. 11

## Fig. 12

## Fig. 13

W20/17
(2E5cells/200µl)

Glass ring

Sample

Side view    Top view

5ml of
medium
from the
side after
4h

**Fig. 14**

**Fig. 15**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6491723 B
- WO 2005055860 A
- US 6183255 B
- US 20030175444 A1
- US 2003099762 A
- US 5571188 A
- US 5480438 A
- WO 2006107336 A
- US 20060193968 A
- WO 2007054505 W

### Non-patent literature cited in the description

- **RATNER et al.** Biomaterials Science; An Introduction to Material in Medicine. 2004
- **BRUNETTE et al.** Titanium is Medicine. 2001
- **ELLINGSEN et al.** Bio-Implant Interface: Improving Biomaterials and Tissue Reaction. CRC Press, 2003
- **LIN et al.** Corrosion test, cell behavior test, and in vivo study of gradient TiO2 layers produced by compound electrochemical oxidation. *Journal of Biomedical Materials Research,* 01 September 2006, vol. 78 (3), 515-22
- **YANG et al.** A review on calcium phosphate coatings produced using a sputtering process-an alternative to plasma spraying. *Biomaterials,* 2005, vol. 26, 327-337
- Bioceramics: material characteristics versus in vivo behaviour. **DUCHEYNE et al.** Annuls of the New York Academy of Science. 10 June 1988, 523
- **HENCH.** Biomaterials: a forecast for the future. *Biomaterials,* 1998, vol. 19, 1419-14239
- **KOKUBO et al.** How useful is SBF in predicting in vivo bone bioactivity. *Biomaterials,* 2006, vol. 27, 2907-2915
- **DE AZA et al.** Bioactivity of pseudowollastonite in human saliva. *Journal of Dentistry,* 1999, vol. 27, 107-113
- **KIM.** Ceramic bioactivity and related biomimetic strategy. *Currant Opinion in Solid State & Materials Science,* 2003, vol. 7, 289-299
- **MA et al.** Biomimetic processing of nanocrystallite bioactive apatite coating on titanium. *Nanotechnology,* 2003, vol. 14, 619-623
- **ZHOU et al.** Plasma-controlled nanocrystallinity and phase composition of TiO2: a smart way to enhance biomimetic response. *J. Biomedical Materials Research,* 2007, vol. 81A, 453-464
- **ROSSI et al.** Comparison between sol-gel-derived anatase- and rutile-structured TiO2 coatings in soft-tissue environment. *J. Biomedical Materials Research,* 2007, vol. 82A, 965-974
- **CULLITY.** Elements of X-ray Diffraction. Addison-Wesley, 1978
- The Scherrer Formula for X-Ray Particle Size Determination. *Phys. Rev. (American Physical Society),* November 1939, vol. 56 (10), 978-982
- **CULLITY et al.** Elements of X-ray Diffraction. 2001
- **SAFI.** Recent aspects concerning DC reactive magnetron sputtering of thin films: a review. *Surface and Costings Technology,* 2000, vol. 127, 203-219
- **GUERIN et al.** Reactive-sputtering of titanium oxide thin films. *Journal of Vacuum Science & Technology A,* 1997, vol. 15 (3), 712-715
- **BAROCH et al.** Reactive magnetron sputtering of Ti-Ox films. *Surface & Coatings Techrrology,* 2005, vol. 193, 107-111
- **BARNES et al.** The mechanism of TiO2 deposition by direct current magnetron reactive sputtering. *Thin Solid Films,* 2004, vol. 446, 29-36
- **BARNES et al.** The mechanism of low temperature deposition of crystalline anatase by DC magnetron sputtering. *Sulface & Costings Technology,* 2005, vol. 190, 321-330
- **KOKUBO et al.** How useful is SBF in predicting in vivo bone bioactivity?. *Biomaterials,* 2006, vol. 27, 2907-2915
- **MCLEOD et al.** Adsorption of bisphosphonate onto hydroxyapatite using a novel co-precipitation technique for bone growth enhancement. *J. Biomedical Materials Research,* November 2006, vol. 79A (2), 271-281